(19) Europäisches Patentamt
European Patent Office
Office européen des brevets

(11) EP 1 660 592 B1

(12) **EUROPÄISCHE PATENTSCHRIFT**

(45) Veröffentlichungstag und Bekanntmachung des Hinweises auf die Patenterteilung:
**19.10.2016 Patentblatt 2016/42**

(21) Anmeldenummer: **04763094.2**

(22) Anmeldetag: **05.07.2004**

(51) Int Cl.:
***C09C 1/36*** *(2006.01)* ***C09C 3/06*** *(2006.01)*
***A61Q 17/04*** *(2006.01)* ***B82Y 5/00*** *(2011.01)*
***B82Y 30/00*** *(2011.01)* ***A61K 8/29*** *(2006.01)*
***A61K 8/04*** *(2006.01)*

(86) Internationale Anmeldenummer:
**PCT/EP2004/007311**

(87) Internationale Veröffentlichungsnummer:
**WO 2005/019348 (03.03.2005 Gazette 2005/09)**

(54) **NANOPARTIKULÄRES UV-SCHUTZMITTEL MIT SILICIUMDIOXIDBESCHICHTUNG**

SILICON DIOXIDE-COATED NANOPARTICULATE UV PROTECTANT

AGENT ANTI-UV NANOPARTICULAIRE POURVU D'UN REVETEMENT DE DIOXYDE DE SILICIUM

(84) Benannte Vertragsstaaten:
**AT BE BG CH CY CZ DE DK EE ES FI FR GB GR HU IE IT LI LU MC NL PL PT RO SE SI SK TR**

(30) Priorität: **21.07.2003 DE 10333029**

(43) Veröffentlichungstag der Anmeldung:
**31.05.2006 Patentblatt 2006/22**

(73) Patentinhaber:
- **Merck Patent GmbH**
  **64293 Darmstadt (DE)**
- **Sachtleben Chemie GmbH**
  **47198 Duisburg (DE)**

(72) Erfinder:
- **PFLÜCKER, Frank**
  **64297 Darmstadt (DE)**
- **HIRTHE, Bernd**
  **47918 Toenisvorst (DE)**
- **SÄNGER, Heike**
  **47495 Rheinberg (DE)**
- **JOHN, Stephan**
  **47198 Duisburg (DE)**

(56) Entgegenhaltungen:
**EP-A- 0 988 853**
**EP-A- 1 284 277**
**EP-A- 1 287 807**
**EP-A2- 0 595 471**
**WO-A-02/068544**
**DE-A1- 2 740 561**
**US-A- 2 885 366**
**US-A1- 2002 017 221**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft die Herstellung eines speziellen nanopartikulären UV-Schutzmittels, wie in Anspruch 1 beschrieben. -Es ist bekannt, dass durch Lichtstrahlung mit einer Wellenlänge im Bereich von Es ist bekannt, dass durch Lichtstrahlung mit einer Wellenlänge im Bereich von 280 bis 400 nm die menschliche Epidermis gebräunt werden kann und dass Strahlung mit einer Wellenlänge im Bereich von 280 bis 320 nm, die unter Bezeichnung UV-B bekannt ist, Erytheme und Hautverbrennungen hervorruft, die der Ausbildung von natürlicher Bräune abträglich sein können. Die UV-B-Strahlung sollte deshalb ausgefiltert werden.

Es ist ferner bekannt, dass UV-A-Strahlung mit einer Wellenlänge im Bereich von 320 bis 400 nm, die die Haut bräunt, eine Veränderung der Haut hervorrufen kann, insbesondere im Falle von empfindlicher Haut oder Haut, die kontinuierlich Sonnenstrahlung ausgesetzt ist. UV-A-Strahlung bewirkt insbesondere einen Verlust der Elastizität der Haut und das Auftreten von Falten, was zu einer vorzeitigen Alterung führt. Sie begünstigt das Auslösen einer Erythembildung oder verstärkt diese Reaktion bei manchen Personen, und sie kann sogar die Ursache für durch Licht ausgelöste toxische oder allergische Reaktionen sein. Es ist daher wünschenswert, auch die UV-A-Strahlung auszufiltern.

In der Kosmetik wurden bis jetzt zahlreiche organische Sonnenschutzfilter angegeben, welche die schädliche UV-A-Strahlung mehr oder weniger selektiv absorbieren können.

Eine diesbezüglich besonders interessante Gruppe von UV-A-Filtern besteht gegenwärtig aus den Dibenzoylmethanderivaten, insbesondere 4,4'-Methoxy-tert.-butyldibenzoylmethan, die ein starkes intrinsisches Absorptionsvermögen aufweisen. Diese Dibenzoylmethanderivate, die derzeit als im UV-A-Bereich wirksame Filter an sich wohlbekannte Produkte sind, sind insbesondere in den französischen Patentanmeldungen FR-A-2 326 405 und FR-A-2 440 933 sowie in der europäischen Patentanmeldung EP-A-0114 607 beschrieben. Das 4,4'-Methoxy-tert.-butyldibenzoylmethan ist im Übrigen gegenwärtig unter der Handelsbezeichnung Eusolex® 9020 von der Fa. Merck im Handel erhältlich.

Diese Dibenzoylmethanderivate können mit einem UV-B-Filter kombiniert werden, um einen vollständigen Schutz über das gesamte Spektrum des Sonnenlichts im UV-Bereich zu erhalten.

Es ist ferner bekannt, dass durch die Zugabe eines anorganischen Pigments und insbesondere eines Pigments von Titandioxid ($TiO_2$) die Lichtschutzeigenschaften der Sonnenschutzmittel, die UV-Filter enthalten, verbessert werden können.

Deshalb ist die Kombination von Dibenzoylmethanderivaten und nanopartikuläre Metalloxiden von Metalloxiden auf dem Gebiet der Sonnenschutzmittel sehr geschätzt.

Es zeigt sich aber, dass die Kombinationen von Dibenzoylmethanderivaten und anorganischen nanopartikuläre Metalloxiden und insbesondere die Kombination von 4,4'-Methoxy-tert.-butyldibenzoylmethan und Metalloxiden mehrere Nachteile aufweisen, die sich nicht nur auf die Art und damit die Qualität der Produkte, die sie enthalten, sondern auch auf ihre Attraktivität für die Verbraucher auswirkt. Bei den Zusammensetzungen, die diesen Typ von Kombination enthalten, wird zum einen häufig ein verstärkter Abbau von Dibenzoylmethanderivaten in Formulierungen beobachtet, wenn Titandioxid-Partikel anwesend sind Zum anderen entsehen in kosmetischen Formulierungen, welche diese Kombination enthalten immer wieder Schwierigkeiten durch Auskristallisation von komplexen des Dibenzoylmethanderivats. Weiter wird häufig eine Farbänderung beobachtet, die in einer mehr oder weniger intensiven Gelb- oder Rotfärbung der Formulierungen zum Ausdruck kommt. Abgesehen davon, dass dieses Phänomen das Lichtschutzvermögen der Dibenzoylmethanderivate und insbesondere des 4,4'-Methoxy-tert.-butyldibenzoylmethan vermindert, ist diese Färbung aus kosmetischer Sicht natürlich nicht wünschenswert.

Es wird ferner beobachtet, dass diese Phänomene im Falle der Nanopigmente von $TiO_2$ besonders ausgeprägt sind.

Im Stand der Technik wurden bereits verschiedene Versuche, einzelne dieser Probleme zu lösen, angegeben: In der japanischen Patentanmeldung JP61-215314 wurde die Verwendung von Maskierungsmitteln empfohlen, welche unter Edetinsäure, Metaphosphorsäure, Polyphosphorsäure und/oder den Salzen dieser Säuren ausgewählt sind, um das Phänomen der Gelbfärbung zu vermindern. Diese Lösung ist jedoch nicht völlig zufriedenstellend.

In der Europäischen Patentanmeldung EP-A-0 748 624 wird festgestellt, dass die Verwendung von nanopartikuläre Metalloxiden von Titandioxid, die mit einem Silicon (Silanderivat oder Siloxanderivat) behandelt sind, die Gelbfärbung, die üblicherweise bei den Sonnenschutzmitteln beobachtet wird, welche herkömmliche Kombinationen vom Typ Dibenzoylmethanderivat/Pigment von $TiO_2$ enthalten, deutlich verringert.

Weiter sind aus der Druckschrift WO-A-94/04131 gegenüber Licht stabile Filterzusammensetzungen bekannt, die in wohldefinierten Mengenanteilen ein Dibenzoylmethanderivat in Kombination mit einem Benzylidencampherderivat enthalten. Nach dieser Druckschrift kann das Dibenzoylmethanderivat durch den Benzylidencampher in den angegebenen Mengenanteilen gegenüber Licht stabilisiert, d.h. seine Zersetzung unter der Einwirkung von UV-Strahlung und insbesondere UV-A-Strahlung eingeschränkt werden. In der gleichen Druckschrift ist angegeben, dass diese photostabilen Zusammensetzungen ferner ein organisches Pigment, das UV-Strahlung abblockt, und insbesondere ein Pigment von Titandioxid enthalten können, das mit einer Verbindung und insbesondere mit einer siliconhaltigen Verbindung umhüllt sein kann.

Trotz dieser Versuche, die oben genannten Probleme bei Kombination von Dibenzoylmethanderivaten mit Metalloxid-Partikeln zu lösen, besteht nach wie vor Bedarf nach einer Metalloxid-Qualität, die alle genannten Probleme gleichzeitig in befriedigender Weise löst und nach geeigneten Herstellverfahren.

EP 1287807 beschreibt eine kosmetische Zusammensetzung enthaltend nanopartikuläres Titandioxid, welches mit Siliciumdioxid beschichtet ist und die Verwendung in Sonnenschutzmitteln.

EP 1284277 beschreibt mit Siliciumdioxid umhüllte Metalloxidpartikel.

EP 0988853 beschreibt eine kosmetische Zusammensetzung enthaltend ein mit Siliciumdioxid beschichtetes, nanopartikuläres Metalloxid und ein Verfahren zur Herstellung des nanopartikulären Metalloxids.

US 2002/017221 beschreibt ein Verfahren zur Herstellung von Titandioxidpigmenten, die mit Siliciumdioxid beschichtet sind.

WO 02/068544 beschreibt ein Verfahren zur Herstellung von nanopartikulärem Titandioxid, bei dem eine wässrige Dispersion auf 60-70°C oder 85°C erhitzt wird.

EP 0595471 beschreibt ein Verfahren zur Herstellung von nanopartikulärem Titandioxid, bei dem eine wässrige Dispersion auf 60°C oder 90°C erhitzt wird.

US 2885366 beschreibt ein Verfahren zur Beschichtung von Titandioxidpartikeln mit Kieselsäure, bei dem eine wässrige Dispersion von Titandioxidpartikeln auf 95°C oder 100°C erhitzt wird.

Jetzt wurde überraschend gefunden, dass es möglich ist, bestimmte nanopartikuläre UV-Schutzmittel, die eine Siliciumdioxid-Beschichtung aufweisen herzustellen, die die genannten Probleme in befriedigender Weise lösen.

Ein erster Gegenstand der vorliegenden Erfindung ist ein Verfahren zur Erstellung eines nanopartikulären UV-Schutzmittels, das eine Siliciumdioxid-Beschichtung aufweist, durch hydrothermale Behandlung eines nanopartikulären Metalloxids und anschließendes Aufbringen einer Siliciumdioxid-Beschichtung gemäß Anspruch 1

Als Hydrothermalbehandlung wird das Erhitzen einer wässrigen Lösung, bzw. Suspension oder Dispersion in einem geschlossenen Behälter, gegebenenfalls unter Druck, bezeichnet (vgl. auch Ullmanns Enzyklopädie der Technischen Chemie, 4. Auflage, 1978, Band 15, S.117 ff: K.Recker, Einkristallzüchtung).

Unter einem nanopartikulären UV-Schutzmittel wird im Sinne der vorliegenden Erfindung vorzugsweise ein nanopartikuläres Metalloxid mit Siliciumdioxid-Beschichtung verstanden. Die Kristallitgröße des nanopartikulären Metalloxides in dem nanopartikulären UV-Schutzmittel bestimmt nach der Scherrer-Methode liegt üblicherweise im Bereich von 5 nm bis 100 nm, vorzugsweise im Bereich 8 bis 50 nm und insbesondere bevorzugt unterhalb von 25 nm. Die im Transmissionselektronenmikroskop ermittelbaren Abmessungen des nanopartikulären Metalloxides liegen üblicherweise bei einer Länge von 5 bis 150 nm und einer Breite von 5 bis 60 nm. Vorzugsweise liegt die Länge im Bereich von 20 bis 60 nm und die Breite im Bereich von 8 bis 30 nm.

Als nanopartikuläre Metalloxide kommen dabei für die erfindungsgemäße Verwendung insbesondere Titandioxid, Eisenoxide, Zinkoxid oder auch Ceroxide zum Einsatz, wobei Titandioxid als Metalloxid erfindungsgemäß insbesondere bevorzugt ist. Titandioxid kann dabei in Form von Rutil oder Anatas oder in amorpher Form, vorzugsweise aber in Form von Rutil und/oder Anatas, vorliegen. Bevorzugte Primärpartikelgröße liegt im Bereich vom 5 bis 50 nm. Dabei sind die Primärpartikel insbesondere bei Anatas vorzugsweise rund, während Rutil-Primärpartikel häufig in Nadel- oder Spindelform bis hin zu Ovalen ("eiförmig") auftreten. Es können erfindungsgemäß jedoch auch runde Rutil-Primärpartikel eingesetzt werden.

Die Siliciumdioxid-Beschichtung soll das nanopartikulären Metalloxid möglichst vollständig bedecken, und da es als UV-Filter jedoch inert ist, dennoch nicht in zu großen Mengen vorliegen. Es hat sich gezeigt, dass es vorteilhaft ist, wenn der Siliciumdioxidgehalt bezogen auf das gesamte nanopartikuläre UV-Schutzmittel 5 bis 50 Gew.-%, vorzugsweise 8 bis 30 Gew.-% und insbesondere bevorzugt 12 bis 20 Gew.-% beträgt.

Das resultierende nanopartikuläre UV-Schutzmittel zeigt üblicherweise eine Partikelgröße nach der Scherrer-Methode im Bereich von 5 nm bis 100 nm, vorzuzugsweise im Bereich 8 bis 50 nm und insbesondere bevorzugt unterhalb von 25 nm. Die im Transmissionselektronenmikroskop ermittelbaren Abmessungen des nanopartikulären UV-Schutzmittels liegen üblicherweise bei einer Länge von 5 bis 160 nm und einer Breite von 10 bis 70 nm. Vorzugsweise liegt die Länge im Bereich von 30 bis 70 nm und die Breite im Bereich von 18 bis 40 nm.

Das nanopartikuläre UV-Schutzmittel zeigt dabei vorteilhafte Eigenschaften hinsichtlich:

- UV-Absorption, insbesondere Breitband- bzw. UV-B-Absorption,
- Transparenz im sichtbaren Licht (VIS),
- gute, insbesondere erhöhte Photostabilität,
- verringerte bzw. verhinderte Photoaktivität,
- hydrophile Oberfläche, gute Einarbeitung und Absetzstabilität in wässrigen Phasen;
- Silica-Oberfläche, die ggf. leicht, mit bekannten Techniken hydrophob modifiziert werden kann,
- leichte Dispergierbarkeit in wässrigen und öligen Phasen,
- in Kombinaton mit Dibenzoylmethan-Derivaten, insbesondere:

- ◦ verringerte Verfärbung der Formulierung und/oder
- ◦ nachlassende Verfärbung der Formulierung während der Lagerung und/oder
- ◦ keine oder reduzierte Auskristallisation von Komplexen der Dibenzoylmethan-Derivate und/oder
- ◦ erhöhte Lagerstabilität der Dibenzoylmethan-Derivate und/oder
- ◦ verbesserte Lichtschutzwirkung, insbesondere nach Lagerung,

- in Kombination mit Selbstbräunern, insbesondere Dihydoxyaceton, wird keine oder eine gegenüber dem stand der Technik verminderte Destabilisierung des Selbstbräuners beobachtet,
- in Kombination mit Benzophenon-Derivaten, insbesondere 2-Hydroxy-4-Methoxy-Benzophenon, wird eine Stabilisierung der Benzophenon-Derivate beobachtet.

Dabei hat es sich insbesondere gezeigt, dass es zur gleichzeitigen Verwirklichung der oben genannten Eigenschaften vorteilhaft sein kann, wenn das nanopartikuläre Metalloxid mit Cer oder Eisen, vorzugsweise Eisen, dotiert ist.
In einer anderen ebenfalls bevorzugten Variante ist das nanopartikuläre Metalloxid jedoch frei von Dotierstoffen.
Die nachlassende Verfärbung der Formulierung während der Lagerung bei Kombination mit Dibenzoylmethanderivaten zeigt sich dabei bei allen üblichen Lagertemperaturen für kosmetische Formulierungen, insbesondere bei 4°C, Raumtemperatur und 50°C. Diese positive Wirkung beginnt direkt nach Herstellung der Formulierung. Eine erneute Intensivierung der Verfärbung tritt - soweit bislang bekannt - in der üblichen Lebensdauer einer kosmetischen Formulierung nicht ein.
Wie bereits oben erwähnt, werden die nanopartikulären UV-Schutzmittel mit den beschriebenen Eigenschaften erhalten, wenn das Herstellverfahren nach Anspruch 1 eingehalten wird.
Entsprechend ist das Verfahren nach Anspruch 1 zur Herstellung eines nanopartikulären Metalloxids mit Lichtschutzeigenschaften , das dadurch gekennzeichnet ist, dass

a) ein nanopartikuläres Metalloxid hydrothermal behandelt wird und
b) anschließend eine Siliciumdioxid-Beschichtung aufgebracht wird,

der Gegenstand der vorliegenden Erfindung.
Wie bereits oben ausgeführt kann es bei diesem Verfahren bevorzugt sein, wenn das in Schritt a) eingesetzte nanopartikuläre Metalloxid ein nanopartikuläres Titandioxid ist, das vorzugsweise mit Eisen dotiert sein kann.
Die Hydrothermalbehandlung wird dabei bei Temperaturen im Bereich von 140 bis 360°C, vorzugsweise im Bereich von 140 bis 200°C, durchgeführt-.
Durch die Hydrothermalhehandlung kommt es zur Ausbildung stabiler Nano-Kristallite mit gleichmäßiger Größe und Form. Bei niedrigen Temperaturen entstehen "nadelförmige" Kristallite. Mit zunehmende Temperatur verrunden die Kristallite. Es bilden sich ovale Fomen, die bis zu runden Partikeln bei sehr hohen Temperaturen gehen. Zusätzlich kommt es zu einem gleichmäßigen Kristallwachstum, was zu einer Erniedrigung der Reaktivität und Photoaktivität führt.
Vorteile der Hydrothermalbehandlung gegenüber einer üblichen thermischen Behandlung (Temperaturbehandlung eines getrockneten Pulvers)- sind-:

- Ausbildung gleichmäßiger Kristallitgrößen mit enger Kornverteilung
- Verhinderung von Sintereffekten (Bildung von unerwünschten Aggregaten)

Die Siliciumdioxid-Beschichtung in Schritt b) wird vorzugsweise als Sol-Gel-Prozess durchgeführt, wobei insbesondere bevorzugt eine Wasserglaslösung zu einer Suspension des Metalloxids gegeben wird.
Dabei wird der Sol-Gel-Prozeß in einer vorteilhaften Variante der vorliegenden Erfindung bei konstant gehaltenem pH-Wert durchgeführt. Der konstant gehaltene pH-Wert kann in einem Bereich von pH 2 bis pH 11 liegen, wobei der pH-Wert vorzugsweise im Bereich von pH = 5 bis pH = 8, insbesonders bevorzugt im Bereich von pH = 6 bis pH = 7 liegt.
Eine weitere vorteilhafte Variante der vorliegenden Erfindung ist die Gesamtzugabe des zur Nachbehandlung notwendigen Wasserglases bei einem pH = 7 bis pH = 11 ohne pH-Konstanthaltung. Anschließend wird der pH-Wert auf einen Wert von pH = 5 bis pH = 8, vorzugsweise auf pH = 6 bis pH = 7 abgesenkt.
Weiter ist es bevorzugt, wenn Schritt b) bei erhöhter Temperatur, vorzugsweise bei einer Temperatur im Bereich von 50°C bis -110°C durchgeführt wird.
Bei allen genannten Varianten des erfindungsgemäßen Verfahrens ist eine Reifezeit nach beendeter Beschichtung vorteilhaft. Die Reifezeit sollte zwischen 1 h und 8 h-, bevorzugt 2 h bis 4 h betragen und bei einer Temperatur von 50°C bis 110°C durchgeführt werden.
Weiter kann es im Hinblick auf die bei der späteren Verarbeitung erwünschten Agglomeratgrößen von Vorteil sein, wenn das Produkt nachträglich vermahlen wird. Hier können die üblichen bei nanopartikulären Materialen verwendbaren Mahltechniken eingesetzt werden.

Aufgrund der oben genannten Vorteile ist das Verfahrensprodukt geeignet, in einer Zubereitung mit Lichtschutzeigenschaften eingesetzt zu werden.

Bei den Zubereitungen handelt es sich vorzugsweise um topisch anwendbare Zubereitungen, beispielsweise kosmetische oder dermatologische Formulierungen. Die Zubereitungen enthalten in diesem Fall einen kosmetisch oder dermatologisch geeigneten Träger und je nach gewünschtem Eigenschaftsprofil optional weitere geeignete Inhaltsstoffe.

Weitere bevorzugte Zubereitungen sind aus der Gruppe Fasern, Textilien, einschließlich deren Beschichtungen, Anstrichstoffe, Beschichtungssysteme, Folien und Verpackungen für den Schutz von Lebensmittel, Pflanzen oder technischen Gütern ausgewählt.

Neben den bereits oben genannten Vorteilen kann der Einsatz der erfindungsgemäß hergestellten nanopartikulären UV-Schutzmittel in Zubereitungen, die Emulsionen sind, insbesondere auch zur Stabilisierung der Emulsion beitragen.

Dadurch kann in der Regel der Einsatz von Emulgatoren verringert werden oder im Einzelfall (Pickering-Emulsion), sogar ganz auf den Einsatz von Emulgatoren verzichtet werden.

Bevorzugte Zubereitungen mit Lichtschutzeigenschaften enthalten dabei mindestens ein Dibenzoylmethanderivat. Dibenzoylmethanderivate sind, wie bereits gezeigt, an sich bereits wohlbekannte Produkte, die insbesondere in den oben genannten Druckschriften FR-A-2 326 405, FR-A-2 440 933 und EP-A-0 114 607 beschrieben sind.
Die Dibenzoylmethanderivate können insbesondere unter den Dibenzoylmethanderivaten der folgenden Formel ausgewählt sein:

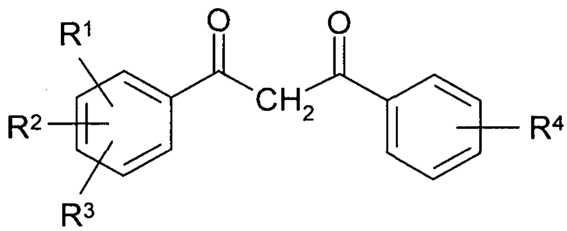

worin $R^1$, $R^2$, $R^3$ und $R^4$, die identisch oder voneinander verschieden sind, Wasserstoff, eine geradkettige oder verzweigte $C_{1-8}$-Alkylgruppe oder eine geradkettige oder verzweigte $C_{1-8}$-Alkoxygruppe bedeuten. Es können selbstverständlich ein Dibenzoylmethanderivat oder mehrere Dibenzoylmethanderivate verwendet werden. Von den Dibenzoylmethanderivaten können insbesondere:

- 2-Methyldibenzoylmethan,
- 4-Methyldibenzoylmethan,
- 4-Isopropyldigenzoylmethan,
- 4-tert.-Butyldibenzoylmethan,
- 2,4-Dimethyldibenzoylmethan,
- 2,5-Dimethyldibenzoylmethan,
- 4,4'-Diisopropyldibenzoylmethan,
- 4,4'-Methoxy-tert.-butyldibenzoylmethan,
- 2-Methyl-5-isopropyl-4'-methoxydibenzoylmethan,
- 2-Methyl-5-tert.-butyl-4'-methoxydibenzoylmethan,
- 2,4-Dimethyl-4'-methoxydibenzoylmethan und
- 2,6-Dimethyl-4-tert.-butyl-4'-methoxydibenzoylmethan genannt werden, wobei diese Aufzählung nicht einschränkend ist.

Von den obengenannten Dibenzoylmethanderivaten wird insbesondere das 4,4'-Methoxy-tert.-butyldibenzoylmethan und insbesondere das unter der Handelsbezeichnung Eusolex® 9020 von der Firma Merck im Handel befindliche 4,4'-Methoxy-tert.-butyldibenzoylmethan bevorzugt, wobei dieses Filter der folgenden Strukturformel entspricht:

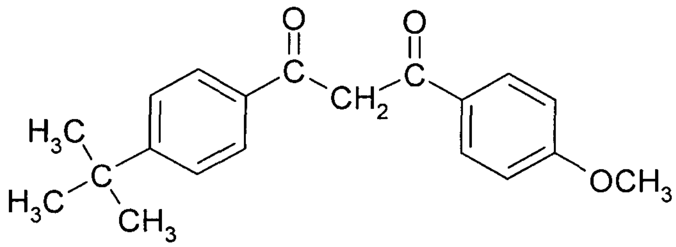

Ein weiteres bevorzugtes Dibenzoylmethanderivat ist das 4-Isopropyldibenzoylmethan.

Weitere bevorzugte Zubereitungen mit Lichtschutzeigenschaften enthalten dabei mindestens ein Benzophenon oder Derivat des Benzophenon, wie insbesondere bevorzugt 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40).

Das oder die Dibenzoylmethanderivat(e) oder das oder die Benzophenonderivat(e) können in den Zusammensetzungen in Mengenanteilen vorliegen, die im Allgemeinen im Bereich von 0,1 bis 10 Gew.-% liegen und vorzugsweise in Mengenanteilen, die im Bereich von 0,3 bis 5 Gew.-% liegen, wobei diese Mengenanteile auf das Gesamtgewicht der Zusammensetzung bezogen sind.

Weiter kann es bevorzugt sein, wenn die Zubereitungen weitere anorganische UV-Filter enthalten. Hierbei sind sowohl solche aus der Gruppe der Titandioxide wie z.B. gecoatetes Titandioxid (z.B. Eusolex® T-2000, Eusolex®T-AQUA), Zinkoxide (z.B. Sachtotec®), Eisenoxide oder auch Ceroxide bevorzugt. Diese anorganischen UV-Filter werden in der Regel in einer Menge von 0,5 bis 20 Gewichtsprozent, vorzugsweise 2 - 10 %, in kosmetische Zubereitungen eingearbeitet. Insbesondere kann es dabei bevorzugt sein, wenn in Emulsionen in einer Phase ein erfindungsgemäß hergestelltes nanopartikulkäres UV-Schutzmittel und in der anderen Phase ein weiterer anorganischer UV-Filter enthalten ist.

In einer weiteren ebenfalls bevorzugten Ausführungsform enthält die Zubereitung mindestens einen Selbstbräuner.

Als vorteilhafte Selbstbräuner können unter anderem eingesetzt werden:

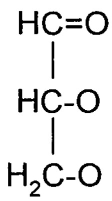

Glycerolaldehyd

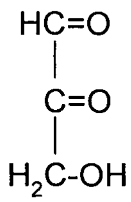

Hydroxymethylglyoxal

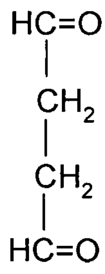

γ-Dialdehyd

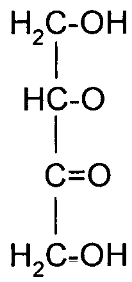

Erythrulose

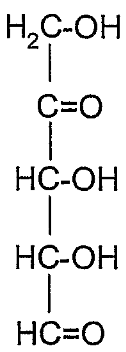

6-Aldo-D-Fructose

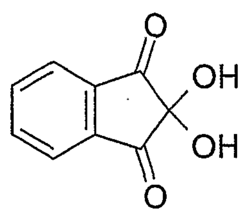

Ninhydrin

Ferner ist das 5-Hydroxy-1,4-naphtochinon (Juglon) zu nennen, das aus den Schalen frischer Walnüsse extrahiert wird

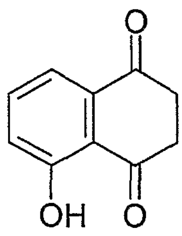

5-Hydroxy-1,4-naphtochinon (Juglon)
sowie das in den Henna-Blättern vorkommende 2-Hydroxy-1,4-naphtochinon (Lawson).

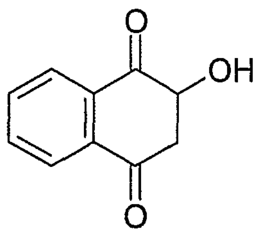

2-Hydroxy-1,4-naphtochinon (Lawson)

Ganz besonders bevorzugt ist das 1,3-Dihydroxyaceton (DHA), ein im menschlichen Körper vorkommender dreiwertiger Zucker und dessen Derivate.

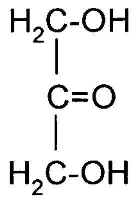

1,3-Dihydroxyaceton (DHA)

Die Verwendung eines erfindungsgemäß hergestellten nanopartikulären UV-Schutzmittels zur Stabilisierung von Selbstbräunern, insbesondere Dihydroxyaceton oder Dihydroxyacetonderivaten ist ebenfalls möglich.

Ferner können die Zubereitungen auch Farbstoffe und Farbpigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramerinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| Chemische oder sonstige Bezeichnung | CIN | Farbe |
|---|---|---|
| Pigment Green | 10006 | grün |
| Acid Green 1 | 10020 | Grün |
| 2,4-Dinitrohydroxynaphthalin-7-sulfonsäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol | 11920 | Orange |
| Solvent Red 3 | 12010 | Rot |

(fortgesetzt)

| Chemische oder sonstige Bezeichnung | CIN | Farbe |
|---|---|---|
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |
| Pigment Brown 1 | 12480 | Braun |
| 4-(2'-Methoxy-5'sulfonsäurediethylamid-1'-phenylazo)-3-hydroxy-5"-chloro-2",4"-dimethoxy2-naphthoesäureanilid | 12490 | Rot |
| Disperse Yellow 16 | 12700 | Gelb |
| 1-(4-Sulfo-1-phenylazo)-4-amino-benzol-5-sulfosäure | 13015 | Gelb |
| 2,4-Dihydroxy-azobenzol-4'-sulfosäure | 14270 | Orange |
| 2-(2,4-Dimethylphenylazo-5-sulfosäure)-1-hydroxynaphthalin-4-sulfosäure | 14700 | Rot |
| 2-(4-Sulfo-1-naphthylazo)-1-naphthol-4-sulfosäure | 14720 | Rot |
| 2-(6-Sulfo-2,4-xylylazo)-1-naphthol-5-sulfosäure | 14815 | Rot |
| 1-(4'-Sulfophenylazo)-2-hydroxynaphthalin | 15510 | Orange |
| 1-(2-Sulfosäure-4-chlor-5-carbonsäure-1-phenylazo)-2-hydroxynaphthalin | 15525 | Rot |
| 1-(3-Methyl-phenylazo-4-sulfosäure)-2-hydroxynaphthalin | 15580 | Rot |
| 1-(4',(8')-Sulfosäurenaphthylazo)-2-hydroxynaphthalin | 15620 | Rot |
| 2-Hydroxy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1 -phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | Rot |
| 1-(2-Sulfo-1-naphthylazo)-2-hydroxynaphthalin-3-carbonsäure | 15880 | Rot |
| 1-(3-Sulfo-1-phenylazo)-2-naphthol-6-sulfosäure | 15980 | Orange |
| 1-(4-Sulfo-1 -phenylazo)-2-naphthol-6-sulfosäüre | 15985 | Gelb |
| Allura Red | 16035 | Rot |
| 1-(4-Sulfo-1 -naphthylazo)-2-naphthol-3,6-disulfosäure | 16185 | Rot |
| Acid Orange 10 | 16230 | Orange |
| 1-(4-Sulfo-1-naphthylazo)-2-naphthol-6, 8-disulfosäure | 16255 | Rot |
| 1-(4-Sulfo-1-naphthylazo)-2-naphthol-3,6,8-trisulfosäure | 16290 | Rot |
| 8-Amino-2-phenylazo-1-naphthol-3,6-disulfosäure | 17200 | Rot |
| Acid Red 1 | 18050 | Rot |
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |
| 4-(4-Sulfo-1-phenylazo)-1 -(4-sulfophenyl)-5-hydroxy-pyrazolon-3-carbonsäure | 19140 | Gelb |
| Pigment Yellow 16 | 20040 | Gelb |
| 2,6-(4'-Sulfo-2",4"-dimethyl)-bis-phenylazo)1,3-dihydroxybenzol | 20170 | Orange |
| Acid Black 1 | 20470 | Schwarz |
| Pigment Yellow 13 | 21100 | Gelb |
| Pigment Yellow 83 | 21108 | Gelb |
| Solvent Yellow | 21230 | Gelb |
| Acid Red 163 | 24790 | Rot |
| Acid Red 73 | 27290 | Rot |
| 2-[4'-(4"-Sulfo-1 "-phenylazo)-7'-sulfo-1'-naphthylazo]-1-hydroxy-7-aminonaphthalin-3,6-disulfosäure | 27755 | schwarz |

(fortgesetzt)

| Chemische oder sonstige Bezeichnung | CIN | Farbe |
|---|---|---|
| 4-[4"-Sulfo-1"-phenylazo)-7'-sulfo-1'-naphthylazo]-1-hydroxy-8-acetyl-aminonaphthalin-3,5-disulfosäure | 28440 | Schwarz |
| Direct Orange 34, 39, 44, 46, 60 | 40215 | Orange |
| Food Yellow | 40800 | Orange |
| trans-ß-Apo-8'-Carotinaldehyd ($C_{30}$) | 40820 | Orange |
| trans-Apo-8'-Carotinsäure ($C_{30}$)-ethylester | 40850 | Orange |
| Canthaxanthin | 40850 | Orange |
| Acid Blue 1 | 42045 | Blau |
| 2,4-Disulfo-5-hydroxy-4'-4"-bis-(diethylamino)triphenyl-carbinol | 42051 | Blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | Grün |
| Acid Blue 7 | 42080 | Blau |
| (N-Ethyl-p-sulfobenzyl-amino)-phenyl-(2-sulfophenyl)-methylen-(N-ethyl-N-p-sulfo-benzyl) $\Delta^{2,5}$-cyclohexadienimin | 42090 | Blau |
| Acid Green 9 | 42100 | Grün |
| Diethyl-di-sulfobenzyl-di-4-amino-2-chlor-di-2-methyl-fuchsonimmonium | 42170 | Grün |
| Basic Violet 14 | 42510 | Violet |
| Basic Violet 2 | 42520 | Violet |
| 2'-Methyl-4'-(N-ethyl-N-m-sulfobenzyl)-amino-4"-(N-diethyl)-amino-2-methyl-N-ethylIN-m-sulfobenzyl-fuchsonimmonium | 42735 | Blau |
| 4'-(N-Dimethyl)-amino-4"-(N-phenyl)-aminonaphtho-N-dimethylfuchsonimmonium | 44045 | Blau |
| 2-Hydroxy-3,6-disulfo-4,4'-bis-dimethylaminonaphthofuchsonimmonium | 44090 | Grün |
| Acid Red 52 | 45100 | Rot |
| 3-(2'-Methylphenylamino)-6-(2'-methyl-4'-sulfophenylamino)-9-(2"-carboxyphenyl)-xantheniumsalz | 45190 | Violet |
| Acid Red 50 | 45220 | Rot |
| Phenyl-2-oxyfluoron-2-carbonsäure | 45350 | gelb |
| 4,5-Dibromfluorescein | 45370 | Orange |
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |
| 2,4,5,7-Tetraiodfluorescein | 45430 | Rot |
| Chinophthalon | 47000 | Gelb |
| Chinophthalon-disulfosäure | 47005 | Gelb |
| Acid Violet 50 | 50325 | Violett |
| Acid Black 2 | 50420 | Schwarz |
| Pigment Violet 23 | 51319 | Violett |
| 1,2-Dioxyanthrachinon, Calcium-Sluminiumkomplex | 58000 | Rot |
| 3-Oxypyren-5,8,10-sulfosäure | 59040 | Grün |
| 1-Hydroxy-4-N-phenyl-aminoanthrachinon | 60724 | Violett |
| 1-Hydroxy-4-(4'-methylphenylamino)-anthrachinon | 60725 | Violett |
| Acid Violet 23 | 60730 | Violett |
| 1,4-Di(4'-methyl-phenylamino)-anthrachinon | 61565 | Grün |
| 1,4-Bis-(o-sulfo-p-toluidino)-anthrachinon | 61570 | Grün |
| Acid Blue 80 | 61585 | Blau |
| Acid Blue 62 | 62045 | Blau |
| N,N'-Dihydro-1,2,1',2'-anthrachinonazin | 69800 | Blau |

(fortgesetzt)

| Chemische oder sonstige Bezeichnung | CIN | Farbe |
|---|---|---|
| Vat Blue 6; Pigment Blue 64 | 69825 | Blau |
| Vat Orange 7 | 71105 | orange |
| Indigo | 73000 | Blau |
| Indigo-disulfosäure | 73015 | Blau |
| 4,4'-Dimethyl-6,6'-dichlorthioindigo | 73360 | Rot |
| 5,5'Dichfor-7,7'-dimethylthioindigo | 73385 | violett |
| Quinacridone Violet 19 | 73900 | violett |
| Pigment Red 122 | 73915 | Rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6, 19; Natural Red 1 | 75100 | gelb |
| Bixin, Nor-Bixin | 75120 | orange |
| Lycopin | 75125 | gelb |
| trans-alpha-, bet- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | Rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | Rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268 :1 | schwarz |
| Chromoxid | 77288 | grün |
| Chromoxid, wasserhaltig | 77278 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyahoferrat | 77510 | blau |
| Pigment White 18 | 77713 | weiß |
| Mangananimoniumdiphosphat | 77742 | violett |
| Manganphosphat; $Mn_3(PO_4)_2 \cdot 7\ H_2O$ | 77745 | rot |
| Silber | 77820 | weiß |

(fortgesetzt)

| Chemische oder sonstige Bezeichnung | CIN | Farbe |
|---|---|---|
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalzem, Anthocyane | | rot |
| Aluminium-, Zink-, Magnesium- und Calciumstearat | | weiß |
| Bromthymolblau | | blau |

Es kann ferner günstig sein, als Farbstoff eine oder mehrere Substanzen aus der folgenden Gruppe zu wählen:

2,4-Dihydroxyazobenzol, 1-(2'-Chlor-4'-nitro-1'phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfonsäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfonsäure, Aluminiumsalz der 4-(4-Sulfo-1-phenylazo)-2-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77492), Manganammoniumdiphosphat und Titandioxid.

Ferner vorteilhaft sind öllösliche Naturfarbstoffe, wie z. B. Paprikaextrakt, β-Carotin oder Cochenille.

Vorteilhaft sind ferner Gelcrèmes mit einem Gehalt an Perlglanzpigmenten. Bevorzugt sind insbesondere die im Folgenden aufgelisteten Arten von Perlglanzpigmenten:

1. Natürliche Perlglanzpigmente, wie z. B.

- "Fischsilber" (Guanin/Hypoxanthin-Mischkristalle aus Fischschuppen) und
- "Perlmutt" (vermahlene Muschelschalen)

2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung/Schichtdicke | Farbe |
|---|---|---|
| **Silberweiße Perlglanzpigmente** | $TiO_2$: 40-60 nm | silber |
| **Interferenzpigmente** | $TiO_2$: 60-80 nm | gelb |
| | $TiO_2$: 80-100 nm | rot |
| | $TiO_2$: 100-140 nm | blau |
| | $TiO_2$: 120-160 nm | qrün |
| **Farbglanzpigmente** | $Fe_2O_3$ | bronze |
| | $Fe_2O_3$ | kupfer |

(fortgesetzt)

| Gruppe | Belegung/Schichtdicke | Farbe |
|---|---|---|
| | $Fe_2O_3$ | rot |
| | $Fe_2O_3$ | rotviolett |
| | $Fe_2O_3$ | rotgrün |
| | $Fe_2O_3$ | schwarz |
| **Kombinationspigmente** | $TiO_2$ / $Fe_2O_3$ | Goldtöne |
| | $TiO_2$ / $Cr_2O_3$ | grün |
| | $TiO_2$ / Berliner Blau | tiefblau |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit $TiO_2$ und $Fe_2O_3$ beschichtete $SiO_2$-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter der Verwendung von $SiO_2$ hergestellt werden. Solche Pigmente-, die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard Mearl auf Basis von Calcium Natrium Borosilikat, die mit Titandioxid beschichtet sind, eingesetzt werden. Diese sind unter dem Namen Reflecks erhältlich. Sie weisen durch ihre Partikelgröße von 40-80 $\mu$m zusätzlich zu der Farbe einen Glitzereffekt auf.

Besonders vorteilhaft sind ferner auch Effektpigmente, welche unter der Handelsbezeichnung Metasomes Standard Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im Allgemeinen verschiedene Farbeffekte hervorgerufen werden. Die Gesamtmenge der Farbstoffe und farbgebenden Pigmente wird vorteilhaft aus dem Bereich von z. B. 0,1 Gew.% bis 30 Gew.%, vorzugsweise von 0,5 bis 15 Gew.%, insbesondere von 1,0 bis 10 Gew.% gewählt, jeweils bezogen auf das Gesamtgewicht der Zubereitungen.

Die nanopartikulären UV-Schutzmittel können auch mit einer die hydrophilen oder die hydrophoben Eigenschaften verstärkenden Oberflächenbehandlung versehen sein. Zur hydropoben Modifikation eignet sich beispielsweise eine Silicon- oder Silan-Beschichtung

Die Silicone sind bekanntlich silicium-organische Polymere oder Oligomere mit geradkettiger oder cyclischer, verzweigter oder vernetzter Struktur mit unterschiedlichen Molekülgewichten, die durch Polymerisation und/oder Polykondensation geeignet funktionalisierter Silane erhalten werden und im Wesentlichen aus wiederkehrenden Haupteinheiten gebildet werden, in denen die Siliciumatome über Sauerstoffatome miteinander verknüpft sind (Siloxanbindung), wobei gegebenfalls substituierte Kohlenwasserstoffgruppen über ein Kohlenstoffatom direkt an die Siliciumatome gebunden sind. Die gebräulichsten Kohlenwasserstoffgruppen sind die Alkylgruppen und inbesondere Methyl, die Fluoralkylgruppen, die Arylgruppen und insbesondere Phenyl sowie die Alkenylgruppen und insbesondere Vinyl. Weitere Typen von Gruppen, die entweder direkt oder über eine Kohlenwasserstoffgruppe an die Siloxankette gebunden werden können, sind insbesondere Wasserstoff, die Halogene und insbesondere Chlor, Brom oder Fluor, die Thiole, die Alkoxygruppen, die Polyoxyalkylengruppen (oder Polyether) und insbesondere Polyoxyethylen und/oder Polyoxypropylen, Hydroxygruppen oder Hydroxyalkylgruppen, die gegebenenfalls substituierten Aminogruppen, die Amidgruppen, die Acyloxygruppen oder Acyloxyalkylgruppen, die Hydroxyalkylaminogruppen oder Aminoalkylgruppen, quaternäre Ammoniumgruppen, amphotere Gruppen oder Betaingruppen, anionische Gruppen, wie Carboxylate, Thioglykolate, Sulfosuccinate, Thiosulfate, Phosphate und Sulfate, wobei diese Aufzählung selbstverständlich in keiner Weise einschränkend ist (sogenannte 'organomodifizierte' Silicone).

Mit dem Ausdruck 'Silicone' sollen auch die zu ihrer Herstellung benötigten Silane und insbesondere die Alkylsilane eingeschlossen und abgedeckt sein.

Die geeigneten Silicone, die zum Umhüllen der nanopartikulären UV-Schutzmittel verwendet werden können, sind vorzugsweise unter den Alkylsilanen, den Polydialkylsiloxanen und den Polyalkylhydrogensiloxanen ausgewählt. Noch bevorzugter sind die Silicone unter Octyltrimethylsilan, den Polydimethylsiloxanen und den Polymethylhydrogenosiloxanen ausgewählt.

Die erfindungsgemäß hergestellten nanopartikulären UV-Schutzmittel können in den Zusammensetzungen in Mengenanteilen vorliegen, die im Allgemeinen im Bereich von 0,1 bis 50 Gew.-% liegen und vorzugsweise in Mengenanteilen, die im Bereich von 0,5 bis 20 Gew.-% liegen, wobei diese Mengenanteile auf das Gesamtgewicht der Zusammensetzung bezogen sind.

Die Sonnenschutzmittel können selbstverständlich einen oder mehrere zusätzliche(n) hydrophile(n) oder lipophile(n) Sonnenschutzfilter, die im UV-A-Bereich und/oder UV-B-Bereich und(/oder IR und/oder VIS-Bereich (Absorber) wirksam sind, enthalten,. Diese zusätzlichen Filter können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben.

Prinzipiell kommen alle UV-Filter für eine Kombination mit den erfindungsgemäß hergestellten nanopartikulären UV-Schutzmitteln in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.

Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL),

Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),

Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex®-HMS),

4-Aminobenzoesäure und Derivate wie 4-Aminobenzoesäure, 4-(Dimethyl-amino)benzoesäure-2-ethylhexylester (z.B. Eusolex® -6007), ethoxylierter 4-Aminobenzoesäureethylester (z.B. Uvinul® P25),

Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;

und weitere Substanzen wie

- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin (z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden. Insbesondere können vorteilhaft auch organische partikuläre UV-Filter, wie Sie beispielsweise in der Patentanmeldung WO 99/66896 beschrieben sind, mit den erfindunsgsgemäß hergestellten nanopartikulären UV-Schutzmitteln kombiniert werden.

Diese organischen UV-Filter werden in der Regel in einer Menge von 0,5 bis 20 Gewichtsprozent, vorzugsweise 1 - 10 Gew.-%, in kosmetische Formulierungen eingearbeitet.

Weitere geeignete organische UV-Filter sind z.B.

- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- α-(Trimethylsilyl)-ω-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1 H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).

- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),

Organische UV-Filter werden insgesamt in der Regel in einer Menge von 0,5 bis 20 Gewichtsprozent, vorzugsweise 1-15 %, in kosmetische Formulierungen eingearbeitet.

Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-propan-1,3-dion, 4-Isopropyldibenzoylmethan, 2-Hydroxy-4-meth-oxy-benzophenon, Methoxyzimtsäureoctylester, 3,3,5-Trimethyl-cyclo-hexyl-sali-cylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfon-säure sowie ihre Kalium-, Natrium- und Triethanolaminsalze.

Bevorzugte Zubereitungen können auch Verbindungen der Formel I enthalten,

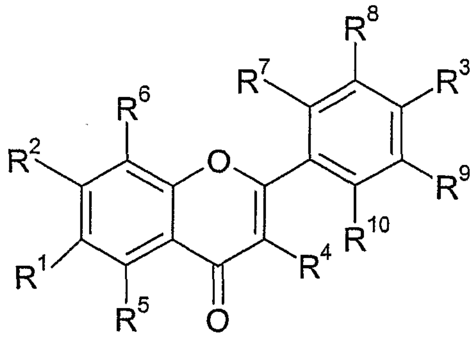

I

wobei $R^1$ und $R^2$ ausgewählt sind aus

- H
- und $OR^{11}$, wobei $OR^{11}$ unabhängig voneinander steht für
  - OH
  - geradkettige oder verzweigte $C_1$- bis $C_{20}$-Alkyloxygruppen,
  - geradkettigen oder verzweigten $C_3$- bis $C_{20}$-Alkenyloxygruppen,
  - geradkettigen oder verzweigten $C_1$- bis $C_{20}$-Hydroxyalkoxy-gruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
  - $C_3$- bis $C_{10}$-Cycloalkyloxygruppen und/oder $C_3$- bis $C_{12}$-Cycloalkenyloxygruppen, wobei die Ringe jeweils auch durch - $(CH_2)_n$-Gruppen mit n = 1 bis 3 überbrückt sein können und/oder,
  - Mono- und/oder Oligoglycosylreste,
    mit der Maßgabe, dass mindestens ein Rest aus $R^1$ und $R^2$ steht für $OR^{11}$, und $R^3$ steht für einen Rest $OR^{11}$ und

$R^4$ bis $R^7$ und $R^{10}$ gleich oder verschieden sein können, und unabhängig voneinander stehen für

- H
- geradkettige oder verzweigte $C_1$- bis $C_{20}$-Alkylgruppen,
- geradkettige oder verzweigte $C_3$- bis $C_{20}$-Alkenylgruppen,
- geradkettige oder verzweigte $C_1$- bis $C_{20}$-Hydroxyalkylgruppen, wobei die Hydroxygruppe an ein primäres, oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können und

$R^8$ und $R^9$ gleich oder verschieden sein können, und unabhängig voneinander stehen für

- H
- $OR^{11}$
- geradkettige oder verzweigte $C_1$- bis $C_{20}$-Alkylgruppen,
- geradkettige oder verzweigte $C_3$- bis $C_{20}$-Alkenylgruppen,

- geradkettige oder verzweigte $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können.

Vorteile der erfindungsgemäßen Zusammensetzungen sind dabei insbesondere die UV-Licht filternde Wirkung und die gute Hautverträglichkeit. Zusätzlich sind die hier beschriebenen Verbindungen der Formel I farblos oder nur schwach gefärbt und führen so, im Gegensatz zu vielen bekannten natürlich vorkommenden Flavonoiden, nicht zu Verfärbungen der Zubereitungen.

Unter den einzusetzbaren Flavonoiden der Formel I finden sich dabei Breitband-UV-Filter. Andere ebenfalls bevorzugte Verbindungen der Formel I zeigen ein Absorptionsmaximum im Grenzbereich zwischen der UV-B- und der UV-A-Strahlung. Als UV-A-II-Filter ergänzen sie daher vorteilhaft das Absorptionsspektrum von handelsüblichen UV-B- bzw. UV-A-I-Filtern. Bevorzugte Zubereitungen mit Lichtschutzeigenschaften enthalten zumindest eine Verbindung der Formel I, wobei $R^3$ steht für

- OH oder
- geradkettige oder verzweigte $C_1$- bis $C_{20}$-Alkoxygruppen, vorzugsweise Methoxy, Ethoxy oder Ethylhexyloxy oder
- Mono- und/oder Oligoglycosylreste, vorzugsweise Glucosylreste und $R^1$ und/oder $R^2$ vorzugsweise stehen für
- OH oder
- geradkettige oder verzweigte $C_1$- bis $C_{20}$-Alkoxygruppen, vorzugsweise Methoxy, Ethoxy oder Ethylhexyloxy oder
- Mono- und/oder Oligoglycosylreste, vorzugsweise Glucosylreste.

Diese bevorzugten Verbindungen zeichnen sich durch eine besonders intensive UV-Absorption aus.

Zusätzlich haben solche bevorzugten Verbindungen Vorteile bei der Einarbeitung in die Zubereitungen:

- Mono- und/oder Oligoglycosylreste verbessern die Wasserlöslichkeit der erfindungsgemäß einzusetzenden Verbindungen;
- geradkettige oder verzweigte $C_1$- bis $C_{20}$-Alkoxygruppen, insbesondere die langkettigen Alkoxyfunktionen, wie Ethylhexyloxy-Gruppen erhöhen die Öllöslichkeit der Verbindungen;

d.h. über die geeignete Auswahl der Substituenten kann die Hydrophilie bzw. Lipophilie der Verbindungen nach Formel I gesteuert werden. Als Mono- oder Oligosaccharidreste bevorzugt sind dabei Hexosylreste, insbesondere Ramnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch vorteilhaft sein, Pentosylreste zu verwenden. Die Glycosylreste können $\alpha$- oder $\beta$-glycosidisch mit dem Grundkörper verbunden sein. Ein bevorzugtes Disaccharid ist beispielsweise das 6-O-(6-deoxy-$\alpha$-L-mannopyranosyl)-$\beta$-D-glucopyranosid.

Es hat sich gezeigt, dass die Intensität der UV-Absorption insbesondere dann hoch ist, wenn $R^3$ steht für geradkettige oder verzweigte $C_1$- bis $C_{20}$-Alkoxygruppen, vorzugsweise Methoxy, Ethoxy oder Ethylhexyloxy, und $R^8$ und $R^9$ gleich sind und stehen für H oder geradkettige oder verzweigte $C_1$- bis $C_{20}$-Alkoxygruppen, vorzugsweise Methoxy, Ethoxy oder Ethylhexyloxy.

Daher sind Zubereitungen mit Lichtschutzeigenschaften enthaltend zumindest eine Verbindung der Formel I, die dadurch gekennzeichnet ist, dass $R^3$ steht für geradkettige oder verzweigte $C_1$- bis $C_{20}$-Alkoxygruppen, vorzugsweise Methoxy, Ethoxy oder Ethylhexyloxy, und $R^8$ und $R^9$ gleich sind und stehen für H oder geradkettige oder verzweigte $C_1$- bis $C_{20}$-Alkoxygruppen, vorzugsweise Methoxy, Ethoxy oder Ethylhexyloxy, besonders bevorzugt. Insbesondere ist es dabei bevorzugt, wenn und $R^8$ und $R^9$ für H stehen.

Die Verbindungen der Formel I werden typisch in Mengen von 0,01 bis 20 Gew.-%, vorzugsweise in Mengen von 0,5 Gew.-% bis 10 Gew.-% und insbesondere bevorzugt in Mengen von 1 bis 8 Gew.-% eingesetzt. Dabei bereitet es dem Fachmann keinerlei Schwierigkeiten die Mengen abhängig von dem beabsichtigten Lichtschutzfaktor der Zubereitung entsprechend auszuwählen.

Durch Kombination von einem oder mehrerer nanopartikulärer UV-Schutzmittel mit weiteren UV-Filtern kann die Schutzwirkung gegen schädliche Einwirkungen der UV-Strahlung optimiert werden. Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten.

Alle genannten UV-Filter einschließlich der Verbindungen der Formel I können auch in verkapselter Form eingesetzt werden. Insbesondere ist es von Vorteil organische UV-Filter in verkapselter Form einzusetzen. Im Einzelnen ergeben sich die folgenden Vorteile:

- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.

- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.

- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.

- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher kann es bevorzugt sein, wenn ein oder mehrere der Verbindungen gemäß Formel I bzw. der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Siliciumoxidhydroxid) aufgebaut sind. Die Herstellung entsprechender Kapseln ist dem Fachmann beispielsweise aus den zitierten Patentanmeldungen bekannt.

Dabei sind die Kapseln in den Zubereitungen vorzugsweise in solchen Mengen enthalten, die gewährleisten, dass die verkapselten UV-Filter in den oben angegebenen Mengen in der Zubereitung vorliegen.

Weisen die Zubereitungen Verbindungen entsprechend Formel I mit freien Hydroxy-Gruppen auf, so zeigen sie neben den beschriebenen Eigenschaften zusätzlich eine Wirkung als Antioxidans und/oder Radikalfänger. Bevorzugt sind daher auch Zubereitungen mit Lichtschutzeigenschaften enthaltend zumindest eine Verbindung der Formel I, die dadurch gekennzeichnet ist, dass mindestens einer der Reste $R^1$ bis $R^3$ steht für OH, wobei vorzugsweise mindestens einer der Reste $R^1$ oder $R^2$ für OH steht.

Damit die Verbindungen der Formel I ihre positive Wirkung als Radikalfänger auf die Haut besonders gut entwickeln können, kann es bevorzugt sein die Verbindungen der Formel I in tiefere Hautschichten eindringen zu lassen. Dazu stehen mehrere Möglichkeiten zur Verfügung. Zum einen können die Verbindungen der Formel I eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der Verbindungen der Formel I durch die äußeren Hautschichten ermöglichen. Schließlich ist auch ein systemischer Transport der Verbindungen der Formel I denkbar. Die Zubereitung wird dann beispielsweise so gestaltet, dass sie für eine orale Gabe geeignet ist.

Allgemein wirken die Substanzen der Formel I als Radikalfänger. Solche Radikale werden nicht nur durch Sonnenlicht erzeugt, sondern werden unter verschiedenen Bedingungen gebildet. Beispiele sind Anoxie, die den Elektronenfluß stromauf der Cytochromoxidasen blockiert und die Bildung von Superoxidradikalarionen bedingt; Entzündungen, die unter anderem mit der Bildung von Superoxidanionen durch die Membran-NADPH-Oxidase der Leukozyten einhergehen, die jedoch auch mit der Bildung (durch Disproportionierung in Gegenwart von Eisen (II)-ionen) der Hydroxyradikale und anderer reaktiver Spezies, die normalerweise beim Phänomen einer Phagocytose beteiligt sind, einhergehen; sowie Lipidautooxidation die im Allgemeinen durch ein Hydroxylradikal initiiert wird und lipidische Alkoxyradikale und Hydroperoxide liefert.

Es wird vermutet, dass bevorzugte Verbindungen der Formel I auch als Enzymhemmer wirken. Sie hemmen vermutlich Histidindecarboxylase, Proteinkinasen, Elastase, Aldosereduktase sowie Hyaluronidase, und ermöglichen daher, die Unversehrtheit der Grundsubstanz vaskulärer Hüllen aufrecht zu erhalten. Ferner hemmen sie vermutlich nicht spezifisch Katechol-O-methyltransferase, wodurch die Menge der verfügbaren Katecholamine und dadurch die Gefäßfestigkeit erhöht wird. Weiter hemmen sie AMP-Phosphodiesterase, wodurch die Substanzen ein Potential zur Hemmung der

Thrombozytenaggregation aufweisen.
Aufgrund dieser Eigenschaften eignen sich die Zubereitungen allgemein zur Immunprotektion und zum Schutz der DNA und RNA. Insbesondere eignen sich die Zubereitungen dabei zum Schutz von DNA und RNA vor oxidativen Angriffen, vor Radikalen und vor Schädigung durch Strahlung, insbesondere UV-Strahlung. Ein weiterer Vorteil der Zubereitungen ist der Zellschutz, insbesondere der Schutz von Langerhans-Zellen vor Schäden durch die oben genannten Einflüsse.

Insbesondere eignen sich bevorzugte Zusammensetzungen auch zur Behandlung von Hautkrankheiten, die mit einer Störung der Keratinisierung verbunden sind, die die Differenzierung und Zellproliferation betrifft, insbesondere zur Behandlung der Akne vulgaris, Akne comedonicä, der polymorphen Akne, der Akne rosaceae, der nodulären Akne, der Akne conglobata, der alters-bedingten Aknen, der als Nebenwirkung auftretenden Aknen, wie der Akne solaris, der medikamenten-bedingten Akne oder der Akne professionalis, zur Behandlung anderer Störungen der Keratinisierung, insbesondere der Ichtyosen, der ichtyosiformen Zustände, der Darrier-Krankheit, der Keratosis palmoplantaris, der Leukoplasien, der leukoplasiformen Zustände, der Haut- und Schleimhautflechten (Buccal) (Lichen), zur Behandlung anderer Hauterkrankungen, die mit einer Störung der Keratinisierung zusammenhängen und eine entzündliche und/oder immunoallergische Komponente haben und insbesondere aller Formen der Psoriasis, die die Haut, die Schleimhäute und die Finger und Zehennägel betreffen, und des psoriatischen Rheumas und der Hautatopien, wie Ekzemen oder der respiratorischen Atopie oder auch der Hypertrophie des Zahnfleisches, wobei die Verbindungen ferner bei einigen Entzündungen verwendet werden können, die nicht mit einer Störung der Keratinisierung zusammenhängen, zur Behandlung aller gutartigen oder bösartigen Wucherungen der Dermis oder Epidermis, die gegebenenfalls viralen Ursprungs sind, wie Verruca vulgaris. Veruca plana, Epidermodysplasia verruciformis, orale Papillomatose, Papillomatosis florida, und der Wucherungen, die durch UV-Strahlung hervorgerufen werden können, insbesondere des Epithelioma baso-cellulare und Epithelioma spinocellulare, zur Behandlung anderer Hautkrankheiten, wie der Dermatitis bullosa und der das Kollagen betreffenden Krankheiten, zur Behandlung bestimmter Augenkrankheiten, insbesondere der Hornhauterkrankungen, zur Behebung oder Bekämpfung der lichtbedingten und der mit dem Älterwerden zusammenhängenden Hautalterung, zur Verminderung der Pigmentierungen und der Keratosis actinica und zur Behandlung aller Krankheiten, die mit der normalen Alterung oder der lichtbedingten Alterung zusammenhängen, zur Vorbeugung vor oder der Heilung von Wunden/Narben der Atrophien der Epidermis und/oder Dermis, die durch lokal oder systemisch angewendete Corticosteroide hervorgerufen werden und aller sonstigen Arten der Hautatrophie, zur Vorbeugung vor oder Behandlung von Störungen der Wundheilung, zur Vermeidung oder Behebung von Schwangerschaftsstreifen oder auch zur Förderung der Wundheilung, zur Bekämpfung von Störungen der Talgproduktion, wie Hypersebhorrhö bei Akne oder der einfachen Seborrhö, zur Bekämpfung von oder Vorbeugung von krebsartigen Zuständen oder vor präkanzerogenen Zuständen, insbesondere der promyelozytären Leukämien, zur Behandlung von Entzündungserkrankungen, wie Arthritis, zur Behandlung aller virusbedingten Erkrankungen der Haut oder anderer Bereiche des Körpers, zur Vorbeugung vor oder Behandlung der Alopecie, zur Behandlung von Hautkrankheiten oder Krankheiten anderer Körperbereiche mit einer immunologischen Komponente, zur Behandlung von Herz-/Kreislauf-Erkran-kungen, wie Arteriosklerose oder Bluthochdruck, sowie des Insulin-unabhängigen Diabetes, zur Behandlung von Hautproblemen, die durch UV-Strahlung hervorgerufen werden.

Die schützende Wirkung gegen oxidativen Stress bzw. gegen die Einwirkung von Radikalen kann weiter verbessert werden, wenn die Zubereitungen ein oder mehrere Antioxidantien enthalten.

In einer Ausführungsform handelt es sich bei der Zubereitung daher um eine Zubereitung zum Schutz von Körperzellen gegen oxidativen Stress, insbesondere zur Verringerung der Hautalterung, dadurch gekennzeichnet, dass sie vorzugsweise ein oder mehrere Antioxidantien enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Tri-hydroxybutyrophenon, Quercitin, Harnsäure und deren Derivate, Mannose und deren Derivate, Zink und dessen Derivate (z.B. ZnO,

$ZnSO_4$), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004).

Die Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Vitamin C, DL-α-Tocopherol, Tocopherol-E-Acetat, Nicotinsäure, Pantothensäure und Biotin.

Die Zubereitungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind Pyrimidincarbonsäuren und/oder Aryloxime.

Pyrimidincarbonsäuren kommen in halophilen Mikroorganismen vor und spielen bei der Osmoregulation dieser Organismen eine Rolle (E. A. Galinski et al., Eur. J. Biochem., 149 (1985) Seite 135-139). Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel II eingesetzt,

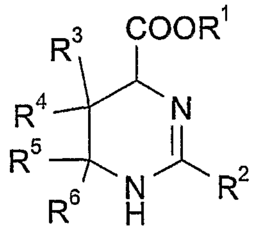

II

worin $R^1$ ein Rest H oder C1-8-Alkyl, $R^2$ ein Rest H oder C1-4-Alkyl und $R^3$, $R^4$, $R^5$ sowie $R^6$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die Zubereitungen derartige Pyrimidincarbonsäuren vorzugsweise in Mengen bis zu 15 Gew.-%.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Zubereitungen, die Aryloxime, vorzugsweise 2-Hydroxy-5-methyllaurophenonoxim enthalten, zeigen überraschende antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew-% Aryloxim enthält.

Alle hier beschriebenen Verbindungen oder Komponenten, die in den Zubereitungen verwendet werden können, sind entweder bekannt und käuflich erwerbbar oder können nach bekannten Verfahren synthetisiert werden.

Neben den hier beschriebenen Verbindungen können die Zubereitungen auch mindestens einen Photostabilisator, vorzugsweise entsprechend der Formel III

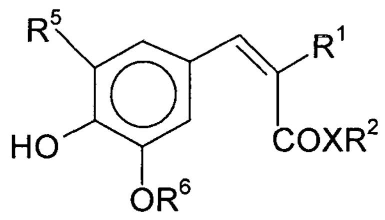

III,

wobei

$R^1$ ausgewählt ist aus $-C(O)CH_3$, $-CO_2R^3$, $-C(O)NH_2$ and $-C(O)N(R^4)_2$;
X is O or NH;
$R^2$ steht für einen linearen oder verzweigten $C_{1-30}$-Alkylrest;
$R^3$ steht für einen linearen oder verzweigten $C_{1-20}$-Alkylrest,
alle $R^4$ unabhängig voneinander stehen für H oder lineare oder verzweigte $C_{1-8}$-Alkylreste
$R^5$ steht für H, einen linearen oder verzweigten $C_{1-8}$-Alkylrest oder einen linearen oder verzweigten $-O-C_{1-8}$-Alkylrest und
$R^6$ steht für einen $C_{1-8}$-Alkylrest,

wobei es sich bei dem Photostabilisator insbesondere bevorzugt um 2-(4-Hydroxy-3,5-dimethoxy-benzyliden)-malonsäure-bis-(2-ethyl-hexyl)ester handelt, enthalten. Entsprechende Photostabilisatoren, ihre Herstellung und Verwendung sind in der Internationalen Patentanmeldung WO 03/007906 beschrieben.

Die Zusammensetzungen können nach Verfahren hergestellt werden, die dem Fachmann gut bekannt sind, insbesondere nach den Verfahren, die zur Herstellung von Öl-in-Wasser-Emulsionen oder Wasser-in-Öl-Emulsionen dienen.

Diese Zusammensetzungen können insbesondere in Form von einfachen oder komplizierten Emulsionen (O/W, W/O, O/W/O oder W/O/W), wie Cremes, Milchen, Gelen oder Gel-Cremes, Pulvern und festen Stiften, vorliegen und gegebenenfalls können sie als Aerosole konfektioniert sein und in Form von Schäumen oder Sprays vorliegen. Vorzugsweise liegen diese Zusammensetzungen in Form einer O/W-Emulsion vor.

Die kosmetischen Zusammensetzungen können als Zusammensetzungen zum Schutz der menschlichen Epidermis oder der Haare gegen UV-Strahlung, als Sonnenschutzmittel oder Schminkprodukte verwendet werden.

Es soll darauf hingewiesen werden, dass in den Formulierungen zum Sonnenschutz, die einen Träger vom Typ einer Öl-in-Wasser-Emulsion aufweisen, die wässerige Phase (die insbesondere die hydrophilen Filter enthält) im Allgemeinen 50 bis 95 Gew.-% und vorzugsweise 70 bis 90 Gew.-%, bezogen auf die gesamte Formulierung, die Ölphase (die insbesondere die lipophilen Filter enthält) 5 bis 50 Gew.-% und vorzugsweise 10 bis 30 Gew.-%, bezogen auf die gesamte Formulierung und der (Co)emulgator oder die (Co)emulgatoren 0,5 bis 20 Gew.-% und vorzugsweise 2 bis 10 Gew.-%, bezogen auf die gesamte Formulierung, ausmachen.

Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten Zubereitungsformen gehören insbesondere Emulsionen.

Emulsionen sind vorteilhaft und enthalten z. B.- die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettlkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersiönen oder Lipodispersionen wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden

Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Emulsionen sind vorteilhaft und enthalten z. B. die genannten Fette, Öle, Wachse und anderen Fettkörper, sowie Wasser und einen Emulgator, wie er üblicherweise für einen solchen Typ der Formuierung verwendet wird.

In einer bevorzugten Ausführungsform enthalten die Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

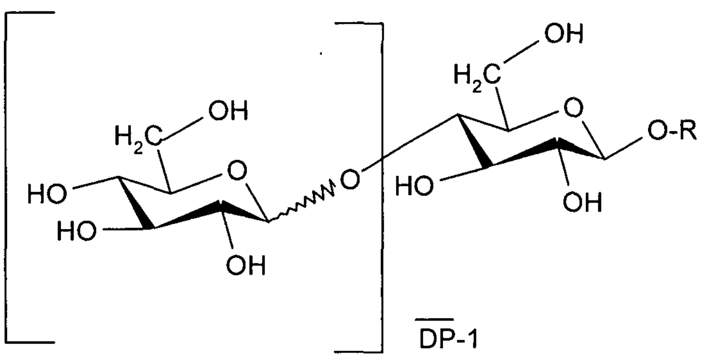

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet.

Der Wert $\overline{DP}$ repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + \ldots = \sum \frac{p_i}{100} \cdot i$$

Dabei stellen $p_1$, $p_2$, $p_3$ ... bzw. $p_i$ den Anteil der einfach, zweifach dreifach ... i-fach glucosylierten Produkte in Gewichtsprozenten dar. Vorteilhaft werden Produkte mit Glucosylierungsgraden von 1-2, insbesondere vorteilhaft von 1, 1 bis 1,5, ganz besonders vorteilhaft von 1,2-1,4, insbesondere von 1,3 gewählt.

Der Wert DP trägt dem Umstande Rechnung, dass Alkylglucoside herstellungsbedingt in der Regel Gemische aus Mono- und Oligoglucosiden darstellen. Vorteilhaft ist ein relativ hoher Gehalt an Monoglucosiden, typischerweise in der Größenordnung von 40-70 Gew.-%.

Besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

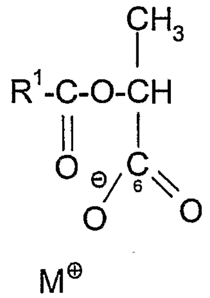

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der Gesellschaft American Ingredients Company.

Die Betaine werden vorteilhaft gewählt aus der Gruppe der Substanzen, welche sich durch die Strukturformel

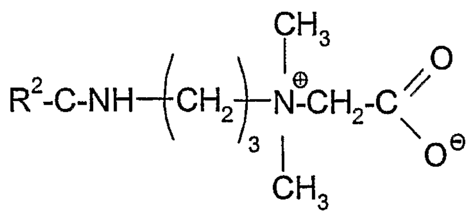

auszeichnen, wobei $R^2$ einen verzweigten oder unverzeigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die Zubereitungen sind vorteilhaft dadurch gekennzeichnet, dass das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Zu Anwendung werden die kosmetischen und dermatologischen Zubereitungen in der für Kosmetika üblichen Weise auf die Haut und/oder die Haare in ausreichender Menge aufgebracht.

Kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form

darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-emulgatoren in den bevorzugten O/W-Emulsionen zu verwenden.

Als besonders bevorzugter Emulgator für O/W-Emulsionen hat sich das Handelsprodukt Ceralution C der Firma Sasol erwiesen.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17),Polyethylenglycol(18)stearylether (Steareth-18), Polyethylenglycol(19)stearylether (Steareth-19), Polyethylenglycol(20)-stearylether (Steareth-20), Polyethylenglycol(12)isostearylether (Isosteareth-12), Polyethylenglycol(13)isostearylether (Isosteareth-13), Polyethylenglycol(14)-isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20)isostearylether (Isosteareth-20), Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15), Polyethylenglycol(12)laurylether (Laureth-12), Polyethylenglycol(12)-isolaurylether (Isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)-cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:

Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C--Atomen, Monoglycerinether gesättigter und/oder, ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Bevorzugte Zubereitungen eignen sich besonders zum Schutz menschlicher Haut gegen UV-induzierte Alterungsprozesse sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrig-alkoholischer Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Bevorzugt ist eine Emulsion, welche als Schutzcreme oder -milch vorliegt und außer der oder den Verbindungen der Formel I beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel

der Haare gewählt werden. Die Zubereitung mit Lichtschutzeigenschaften kann verschiedene, in diesem Mitteltyp verwendete Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Im Folgenden wird die Erfindung anhand von Beispielen näher erläutert oder Beispiele von Zubereitungen und deren Eigenschaften gegeben, die die erfindungsgemäß hergestellten UV-Schutzmittel enthalten.

## Beispiele

### Beispiel 1a Herstellung von nano-$TiO_2$

710 mL Natriumtitanat (Gehalt 140 g $Ti0_2$/L-), erhalten durch Umsetzung von Metatitansäure mit Natronlauge, wird mit 100mL Wasser verdünnt und durch Zugabe von Salzsäure unter Bildung von Titandioxid (Rutil) bei pH 2,2 - 2,6 zersetzt. Dieses durch die Zersetzung erhaltene nanopartikuläre Titandioxid wird unter Zugabe von 115 mL 30 %iger Salzsäure peptisiert und durch weitere Wasserzugabe auf 1000 mL-Gesamtvolumen ergänzt. Die Peptisation erfolgt in einem geschlossenen Glaskolben bei 105°C über einem Zeitraum von 2 h. Das Produkt zeigt nadelförmige Kristallite (Fig. 1)

### Beispiel 1b Herstellung von nano-$TiO_2$

Das aus Versuch 1 a erhaltene Produkt wird nach beendeter Peptisation für die Dauer von 2 h einer weiteren Hydrothermalbehandlung in einem Druckbehälter bei einer Temperatur von 180°C unterzogen. Das resultierende Produkt zeigt ovale Kristallite (Fig.2).

### Beispiel 2a: Beschichtung des nano-$TiO_2$ mit $SiO_2$

1 L der wässrigen, salzsauren Suspension des $TiO_2$ aus Beispiel 1 b wird mit NaOH auf einen pH-Wert von 6,5 gebracht und auf 80°C erhitzt. Anschließend werden zu der Suspension 52 mL Wasserglaslösung (entsprechend 384 g $SiO_2$ / L) bei konstantem pH-Wert (pH = 6,5 $\pm$ 0,5; Regelung durch Zugabe von $H_2SO_4$) zugegeben. Nach erfolgter Zugabe wird bei pH = 6,8 und 80°C 2 Stunden gerührt. Das Produkt wird anschließend auf eine Leitfähigkeit kleiner 100 µS/cm gewaschen und getrocknet.

### Beispiel 2b: Beschichtung des nano-$TiO_2$ mit $SiO_2$

1 L der wässrigen, salzsauren Suspension des $TiO_2$ aus Beispiel 1b wird mit NaOH auf einen pH-Wert von 9,0 gebracht und auf 80°C erhitzt. Anschließend werden zu der Suspension 52 mL Wasserglaslösung (entsprechend 384 g $SiO_2$ / L) bei konstantem pH-Wert (pH = 9,0 $\pm$ 0,5, Regelung durch Zugabe von $H_2SO_4$) zugegeben. Nach erfolgter Zugabe wird bei pH = 6,8 und 80°C 2 Stunden gerührt. Das Produkt wird anschließend auf eine Leitfähigkeit kleiner 100 µS/cm gewaschen und getrocknet.

### Beispiel 2c: Beschichtung des nano-$TiO_2$ mit $SiO_2$

1 L der wässrigen, salzsauren Suspension des $TiO_2$ aus Beispiel 1 b wird mit NaOH auf einen pH-Wert von 2,0 gebracht und auf 80°C erhitzt. Anschließend werden zu der Suspension 52 mL Wasserglaslösung (entsprechend 384 g $SiO_2$ / L) bei konstantem pH-Wert (pH = 2,0 $\pm$ 0,5, Regelung durch Zugabe von $H_2SO_4$) zugegeben. Nach erfolgter Zugabe wird bei pH = 6,8 und 80°C 2 Stunden gerührt. Das Produkt wird anschließend auf eine Leitfähigkeit kleiner 100 µS/cm gewaschen und getrocknet.

### Beispiel 2d: Beschichtung des nano-$TiO_2$ mit $SiO_2$

1 L der wässrigen, salzsauren Suspension des $TiO_2$ aus Beispiel 1 b wird mit NaOH auf einen pH-Wert von 9,0 gebracht und auf 80°C erhitzt. Anschließend werden zu der Suspension 52 mL Wasserglaslösung (entsprechend 384g $SiO_2$/L) zugegeben. Der pH- Wert steigt dabei auf ca. 10,6 an. Nach erfolgter Zugabe wird durch Zugabe von Schwefelsäure auf pH 6,5 abgesenkt und danach bei pH = 6,8 und 80°C 2 Stunden gerührt. Das Produkt wird anschließend auf eine Leitfähigkeit kleiner 100 µS/cm gewaschen und getrocknet.

**Beispiel 3: Herstellung einer Lichtschutzzubereitung enthaltend $TiO_2$ und 4,4'-Methoxy-tert.-butyldibenzoylmethan**

Mit folgenden Titandioxid-Qualitäten werden Formulierungen einsprechend Rezepturbeispiel 6 (siehe weiter unten) hergestellt

Beispiel 3a: erfindungsgemäßes Titandioxid nach Beispiel 2a
Beispiel 3b: Titandioxid mit Aluminiumhaltiger Beschichtung (Handelsprodukt MT 100 Z; Fa. Tayca )

In den Formulierungen wird der Gehalt an 4,4'-Methoxy-tert.-butyldibenzoylmethan als Maß für die Lagerstabilität dieser Verbindung nach Lagerung der Formulierungen bei verschiedenen Bedingungen mittels HPLC bestimmt.

Probenvorbereitung:

Ca. 0,1 g der homogenisierten Formulierung werden in einen 100-ml-Messkolben analytisch genau eingewogen und mit ca. 10 ml Wasser dispergiert, anschließend mit Methanol zur Messmarke aufgefüllt.

Faktoreinwaage:

Ca. 30 mg 4,4'-Methoxy-tert.-butyldibenzoylmethan analytisch genau gewogen, in einen 100-ml-Messkolben einwiegen und mit Methanol zur Messmarke auffüllen. 10,0 ml dieser Lösung mit Methanol in einen 100-ml-Messkolben bis zur Messmarke auffüllen. Ist gleich Faktorlösung.

Chromatographische Bedingungen:

| | |
|---|---|
| Säule: | Superspher 100 RP18e; 125-4, Cat. 1.16855 |
| Säulentemperatur: | 25°C |
| Fließmittel: | Methanol/Mischung A (20:80 v/v) 1,5 ml/min.<br>Mischung A: Mischung auf 1 l Ammoniakacetatlösung = 0,005 mol/l und 2 ml Essigsäure 100 %<br>Gradient:<br>Isokratisch |
| Detektion: | Variabler UV-Detektor; 320 nm |
| Dosierung: | 10 µl Dosierschleife |
| Gerät: | z.B. Flüssigchromatograph Hewlett-Packard System 1100 |
| Auswertung: | Flächenauswertung nach der externen Standardmethode. |

Gemessen wurde zu folgenden Zeitpunkten:

A: unmittelbar nach Herstellung der Formulierung
B: nach 4 Wochen Lagerung bei Raumtemperatur im Dunkeln
C: nach 4 Wochen Lagerung bei 5°C im Dunkeln
D: nach 4 Wochen Lagerung bei 40°C im Dunkeln
E: nach 12 Wochen Lagerung bei Raumtemperatur im Dunkeln
F: nach 12 Wochen Lagerung bei 5°C im Dunkeln
G: nach 12 Wochen Lagerung bei 40°C im Dunkeln

Die Ergebnisse sind in Figur 3 dargestellt. Es zeigt sich, dass der Gehalt 4,4'-Methoxy-tert.-butyldibenzoylmethan, im Vergleichsbeispiel 3b bereits nach 4 Wochen Lagerung bei erhöhter Temperatur im Dunkeln reduziert ist, während beim erfindungsgemäßen Beispiel noch kein Abbau nachzuweisen ist. Auch nach 12 Wochen Lagerung verändert sich der Gehalt an 4,4'-Methoxy-tert.-butyldibenzoylmethan im erfindungsgemäßen Beispiel nur wenig, während im Vergleichsbeispiel ein weitgehender Abbau beobachtet wird (40°C im Dunkeln).

Mit den Produkten nach Beispielen 2b, 2c und 2d werden die gleichen Ergebnisse erhalten.

**Beispiel 4: Verfärbungstest in kosmetischen Formulierungen bei UV-Bestrahlung**

Es werden Formulierungen entsprechend Rezepturbeispiel 6 hergestellt. Jeweils eine enthält das erfindungsgemäße Titandioxid nach Beispielen 2a-d, in der Vergleichsprobe wird ein handelsübliches Titandioxid mit Trimethoxyoctylsilan-Coating (Uvinul™ $TiO_2$; Fa. BASF) eingesetzt.

Von den Formulierungen werden jeweils 3 g in eine PMMA Petrischale gegeben und 58 Minuten im Suntest (Suntest CPS mit Xenonstrahler; Solar Standard Filter + Abdeckscheibe zum Probenschutz (Begrenzung der Strahlung auf ≥ 290nm); Radialux mit UV-Sensor) mit maximaler Bestrahlungsstärke 87 $W/m^2$ (UV-Bereich) bestrahlt = 2 MED Hauttyp II (entspricht 500 $J/m^2$).

Die Proben werden vor und nach der Bestrahlung visuell beurteilt:

| | Vorher | nach 2 MED |
|---|---|---|
| Mit Titandioxid nach Beispiel 2a: | weiß | weiß |
| Mit Titandioxid nach Beispiel 2b: | weiß | weiß |
| Mit Titandioxid nach Beispiel 2c: | weiß | weiß |
| Mit Titandioxid nach Beispiel 2d: | weiß | weiß |
| Mit Vergleichssubstanz: | weiß | gelblich |

**Beispiel 5: Kristallbildung in kosmetischer Zubereitung**

Es werden nach unten beschriebener Rezeptur die in der Tabelle genannten Titandioxide in die beschriebenen Formulierung eingearbeitet und direkt nach Herstellung bzw. nach 12 Wochen Lagerung bei Raumtemperatur mikroskopisch untersucht. Die Rezepturen sind 3 Monate lagerstabil bei RT / 5°C / 40°C und im Schaukeltest -5°C / 40°C.

| **Titandioxidtyp** | **Mikroskopie nach Herstellung** | **Mikroskopie nach 12 Wochen Lagerung bei Raumtemperatur im Dunkeln** |
|---|---|---|
| Titandioxid (erfindungsgemäß nach Beispiel 2a) | keine Kristalle | keine Kristalle |
| Titandioxid (aluminiumhaltige Beschichtung) | keine Kristalle | gut sichtbare Kristalle - Länge ca. 10-100 µm |

Verwendetes Mikroskop: Zeiss, Axioskop 2; manuelle Version mit Mikroskopkamera und PC-Kopplung, Objective 10x Ph1, 40x Ph2, 100x pH3; Polarisationsfilter (λ4 Blättchen)

Mit den Produkten nach Beispiel 2b, 2c und 2d werden die gleichen Ergebnisse wie mit Beispiel 2a erhalten.

Rezeptur:

| **Rohstoff (INCI)** | **%** |
|---|---|
| A | |
| Titanium dioxide (Beispiel 2a) | 4,00 |
| Octyl methoxycinnamate | 6,00 |
| Butyl methoxydibenzoylmethane | 1,00 |
| PEG-30 dipolyhydroxystearate | 2,00 |
| PEG-30 dipolyhydroxystearate | 4,00 |
| C 12-15 alkyl benzoate | 6,00 |
| Isohexadecan | 6,00 |
| Cyclomethicone | 2,00 |
| Microcrystalline wax | 2,00 |
| PVP/Eicosene copolymer | 1,00 |
| Tocopheryl acetate | 1,00 |
| B | |
| Glycerin | 3,00 |
| Sodium chloride | 0,40 |
| Propylene glycol (and) diazolidinyl urea and) methylparaben (and) propylparaben | 0,50 |

(fortgesetzt)

| | |
|---|---|
| B | |
| Wasser | 67,10 |

**Herstellung:**

Phase A bis auf Titandioxid zusammen geben und auf 80°C erhitzen. Titandioxid langsam in die heiße Ölphase einrühren und mit dem Handmixer 30 Sekunden Stufe 4 homogenisieren. Phase B auf 80°C erhitzen und langsam unter Rühren zu Phase A geben, bei ca. 60°C 1 Minute mit dem Handmixer Stufe 4 homogenisieren und unter Rühren abkühlen, entlüften.

**Beispiel 6: Verfärbung von kosmetischen Formulierungen beim Lagern**

Mit folgenden Titandioxid-Qualitäten werden Formulierungen einsprechend Rezepturbeispiel 6 hergestellt

Beispiel 6a: erfindungsgemäßes Titandioxid nach Beispiel 2a
Beispiel 6b: Titandioxid mit Aluminiumhaltiger Beschichtung (Handelsprodukt MT 100 Z; Fa. Tayca)
Beispiel 6c: handelsübliches Titandioxid (Handelsprodukt T-805; Fa. Degussa)

Die Formulierungen werden 3 Monate bei 50°C im Dunkeln gelagert. Anschließend werden die Proben in einem Kunstoffprobenhalter mit Quartz-Abdeckung auf einem Farbmessgerät CE7000 (Fa. Gretag-Macbeth) mit einer Bariumsulfat-ausgekleideten Ulbricht-Kugel (Messoptik: diffus; 8°; Lichtart C, Normalbeobachter, ohne Glanz) vermessen. Die Auswertung der Messung erfolgt nach dem L*a*b*-System (CIELab, DIN 6174). Die Messwerte finden sich in folgender Tabelle bzw. in Figur 4.

| Probe | b*-Wert |
|---|---|
| 6a | 3,52 |
| 6b | 5,35 |
| 6c | 10,59 |

Die Verfärbung der Probe 6a mit dem erfindungsgemäßen Titandioxid ist nach 3 Monaten Lagerung deutlich geringer als die Verfärbung der beiden Proben mit handelsüblichen Titandioxid-Qualitäten.

**Rezepturbeispiel 1: Sonnenschutz Softcreme (O/W) SPF 6 (Sun Protection Factor, Colipa-Methode mit 5 Probanden)**

| **Rohstoff (INCI)** | **Gew.-%** |
|---|---|
| A | |
| Produkt aus Beispiel 2a | 3,00 |
| Steareth-10, Steareth-7, Stearyl alcohol | 2,00 |
| Glyceryl stearate, Ceteth-20 | 2,00 |
| Glyceryl stearate | 3,00 |
| Microwax | 1,00 |
| Oleyl oleate | 6,00 |
| Cetearyl octanoate | 14,00 |
| Caprylic/capric triglyceride | 4,00 |
| Propylparaben | 0,05 |
| B | |
| Propylene glycol | 4,00 |
| Allantoin | 0,20 |
| Wasser | 60,60 |

(fortgesetzt)

| | |
|---|---|
| B | |
| Methylparaben | 0,15 |

**Herstellung:**

Phase A und Phase B auf 80 °C erhitzen. Phase B unter Rühren langsam zu Phase A geben, homogenisieren und unter Rühren abkühlen.

**Rezepturbeispiel 2: Sonnenschutz Spray-Lotion (O/W) SPF 18 (Sun Protection Factor, AMA Laboratories, Inc., USA, mit 5 Probanden)**

| Rohstoff (INCI) | % |
|---|---|
| A | |
| Ethylhexyl methoxycinnamate, BHT | 5,00 |
| Produkt aus Beispiel 2b | 4,00 |
| Glyceryl stearate, cetyl alcohol, PEG-75 stearate, ceteth-20, steareth-20 | 3,30 |
| PPG-1-PEG-9 lauryl glycol ether | 0,50 |
| Diisostearoyl trimethylolpropane | 1,50 |
| Siloxy silicate $C_{12-15}$ alkyl benzoate | 3,00 |
| Dioctyl adipate | 4,00 |
| Dimethicone | 2,00 |
| B | |
| Dimethicone copolyol phosphate | 2,50 |
| Butylene glycol | 2,50 |
| Wasser | 70,50 |
| C | |
| PPG-1 Trideceth-6, polyquaternium-37, propylene glycol dicaprylate/dicaprate | 0,47 |
| D | |
| Propylene glycol, DMMDM hydantoin, methylparaben, propylparaben | 0,73 |

**Herstellung:**

Phase A bis auf das Titandioxid zusammengeben und auf 60°C erhitzen. Titandioxid langsam in die geschmolzene Ölphase einarbeiten. Phase B auf 60 °C erhitzen, dann Phase C unter Rühren eindispergieren. Phase A unter hohem Energieeintrag in die Phase B/C einrühren. Unter Rühren abkühlen, und bei 40 °C Phase D zugeben. Homogenisieren und unter Rühren auf 25°C abkühlen.

**Rezepturbeispiel 3: Sonnenschutz Softcreme (O/W) SPF 23 (Sun Protection Factor, Colipa-Methode mit 5 Probanden)**

| Rohstoff (INCI) | % |
|---|---|
| A | |
| Produkt aus Beispiel 2c | 10,00 |
| Steareth-10, steareth-7, stearyl alcohol | 3,00 |
| Glyceryl stearate, ceteth-20 | 3,00 |
| Glyceryl stearate | 3,00 |
| Microwax | 1,00 |
| Oleyl oleate | 4,00 |

(fortgesetzt)

| Rohstoff (INCI) | % |
|---|---|
| A | |
| Cetearyl octanoate | 10,50 |
| Caprylic/capric triglyceride | 4,00 |
| Propylparaben | 0,05 |
| B | |
| Propylene glycol | 4,00 |
| Allantoin | 0,20 |
| Wasser | 57,10 |
| Methylparaben | 0,15 |

**Herstellung:**

Phase A und B auf 80°C erhitzen. Phase B unter Rühren langsam zu Phase A geben, homogenisieren und Unter Rühren abkühlen.

**Rezepturbeispiel 4: Sonnenschutzlotion (O/W)**

| Rohstoff (INCI) | % |
|---|---|
| A | |
| Ethylhexyl methoxycinnamate, BHT | 6,00 |
| Butyl methoxydibenzoylmethane | 1,00 |
| Polyglyceryl-3 methylglucose distearate | 4,00 |
| Ethylhexyl stearate | 8,00 |
| Cetearyl isononanoate | 2,00 |
| PVP/eicosene copolymer | 1,00 |
| Tocopheryl acetate | 1,00 |
| B | |
| Xanthan gum | 0,30 |
| Sodium cetearyl sulfate | 1,00 |
| Glycerin | 5,00 |
| Wasser | 65,70 |
| C | |
| Produkt aus Beispiel 2a | 4,00 |
| D | |
| Phenoxyethanol, butylparaben, ethylparaben, propylparaben, methylparaben | 1,00 |

**Herstellung:**

Phase A auf 80 °C erhitzen. Das Keltrol der Phase B im Wasser vorquellen, dann die restlichen Rohstoffe zugeben und auf 80 °C erhitzen. Phase A zu Phase B gegen und 2 Min. homogenisieren (Stabmixer): unter Rühren abkühlen und bei 35 °C Phase C zufügen. Nochmals 1 Min. homogenisieren (Stabmixer). Auf Raumtemperatur abkühlen und Phase D einrühren.

**Rezepturbeispiel 5: Sonnenschutzlotion (O/W) in vivo SPF 17 ± 3 (Colipa-Methode mit 10 Probanden)**

| Rohstoff (INCI) | % |
|---|---|
| A | |
| Produkt aus Beispiel 2d | 5,00 |
| Ethylhexyl methoxycinnamate, BHT | 5,00 |
| Glyceryl stearate, cetyl alcohol, PEG-75 stearate, ceteth-20, steareth-20 | 3,30 |
| PPG-1-PEG-9 lauryl glycol ether | 0,50 |
| Diisostearoyl trimethylolpropane siloxy silicate | 1,50 |
| C12-15 Alkyl benzoate | 3,00 |
| Dioctyl adipate | 4,00 |
| Dimethicone | 2,00 |
| B | |
| Ectoin | 0,10 |
| Allantoin | 0,20 |
| Dimethicone copolyol phosphate | 2,50 |
| Butylene glycol | 2,50 |
| Wasser | 68,90 |
| C | |
| PPG-1 trideceth-6, polyquaternium-37, propylene glycol dicaprylate/dicaprate | 0,47 |
| D | |
| Propylene glycol, DMMDM hydantoin, ehtylparaben | 0,73 |
| Parfum | 0,30 |

**Herstellung:**

Phase A bis auf das Titandioxid zusammen geben und auf 60 °C erhitzen. Titandioxid langsam in die geschmolzene Ölphase einarbeiten. Phase B auf 60 °C erhitzen, dann Phase C unter Rühren eindispergieren. Phase A unter kräftigem Rühren in die Phase B/C einrühren. Unter Rühren abkühlen und bei 40 °C Phase D zugeben. Homogenisieren und unter Rürhen auf25 °C abkühlen.

**Rezepturbeispiel 6: Sonnenschutzlotion (O/W)**

| Rohstoff (INCI) | % |
|---|---|
| A | |
| Produkt aus Beispiel 3a bzw. 3b | 5,00 |
| Butylmethoxy dibenzoylmethane | 3,00 |
| Steareth-10, steareth-7, stearyl alcohol | 3,00 |
| Glyceryl stearate, ceteth-20 | 3,00 |
| Glyceryl stearate | 3,00 |
| Microwax | 1,00 |
| Oleyl oleate | 4,43 |
| Cetearyl octanoate | 11,64 |
| Caprylic/capric triglyceride | 4,43 |
| Propylparaben | 0,05 |
| B | |
| Propylene glycol | 4,00 |
| Allantoin | 0,20 |
| Wasser | 57,10 |
| Methylparaben | 0,15 |

**Herstellung:**

Phase A und B auf 80 °C erhitzen. Phase B unter Rühren langsam zu Phase A geben, homogenisieren und Unter Rühren abkühlen.

**Rezepturbeispiel 7: Sonnenschutzlotion (O/W) SPF 10 (Sun Protection Factor, Colipa-Methode mit 10 Probanden)**

| | |
|---|---|
| A | |
| Steareth-10, steareth-7, stearyl alcohol | 3,00 |
| Glyceryl stearate, ceteth-20 | 3,00 |
| Cetearyl octanoate | 15,50 |
| Glyceryl stearate | 3,00 |
| Oleyl oleate | 7,00 |
| Microwax | 1,00 |
| Caprylic/capric triglyceride | 6,00 |
| Propylparaben | 0,05 |
| B | |
| 33%-ige wässrige Dispersion des Produktes aus Beispiel 2a | 16,70 |
| Propylene glycol | 4,00 |
| Allantoin | 0,20 |
| Wasser | 40,40 |
| Methylparaben | 0,15 |

**Herstellung:**

Phase A auf 75 °C und Phase B auf 80 °C erhitzen. Phase B langsam in Phase A einrühren. Homogenisieren und unter Rühren abkühlen.

**Rezepturbeispiel 8: Sonnenschutz Spray-Lotion (O/W) SPF 31 (Sun Protection Factor, FDA Methode mit 5 Probanden bei AMA Laboratories)**

| | |
|---|---|
| A | |
| Produkt aus Beispiel 2d | 5,00 |
| Ethylhexyl methoxycinnamate, BHT | 7,50 |
| Benzophenone-3 | 2,50 |
| PEG-100 stearate, glyceryl stearate | 2,80 |
| PPG-1-PEG-9 lauryl glycol ether | 0,40 |
| Dicapryl ether | 4,50 |
| Steareth-10 | 0,50 |
| Stearyl alcohol | 0,60 |
| Dimethicone | 2,00 |
| B | |
| Dimethicone copolyol phosphate | 2,50 |
| Chitosan glycolate | 2,00 |
| Glycerin | 2,50 |
| Wasser | 66,10 |
| C | |
| PPG-1 trideceth-6, polyquaternium-37, propylene glycol dicaprylate/dicaprate | 0,40 |
| D | |
| Propylene glycol, DMMDM hydantoin, methylparaben, propylparaben | 0,70 |

**Herstellung:**

Phase A bis auf das Titandioxid zusammen geben und auf 60 °C erhitzen. Titandioxid langsam in die geschmolzene Ölphase einarbeiten. Phase B-1 auf 60 °C erhitzen, dann Phase B-2 unter Rühren eindispergieren. Phase A unter hohem Energieeintrag in die Phase B einrühren. Unter rühren abkühlen, und bei 40 °C Phase C zugeben. Homogenisieren und unter rühren auf 25 °C abkühlen.

**Rezepturbeispiel 9: Sonnenschutz Creme, hoher SPF, wasserfest (O/W) mit Eusolex® UV-Pearls™ OMC, SPF (in vitro, Diffey-Methode) 64 ± 12, UVA-PF 17**

| | | |
|---|---|---|
| A | | |
| | Wasser | 38,30 |
| | Glycerin | 3,00 |
| | Pentylene glycol | 3,00 |
| | PVP/hexadedecene copolymer | 1,00 |
| | Sodium cetearyl sulfate | 1,00 |
| | Xanthan gum | 0,20 |
| B | | |
| | Glyceryl stearate, cetearyl alcohol, sodium stearoyl lactylate, tocopherol | 5,00 |
| | Tri-C12-13 alkyl citrate | 3,50 |
| | Isopropylphtalimide, butylphtalide | 5,00 |
| | Caprylic/capric triglyceride | 2,50 |
| | C12-15 alkyl benzoate | 2,00 |
| | Cyclomethicone | 0,80 |
| | Tocopheryl acetate | 1,00 |
| | Butyl methoxydibenzoylmethane | 1,00 |
| | Benzophenone-3 | 2,00 |
| | Produkt aus Beispiel 2a | 4,00 |
| C | | |
| | Wasser, ethylhexyl methoxycinnamate, silica, PVP, chlorphenesin, BHT (Eusolex UV Pearl OMC) | 20,00 |
| D | | |
| | Carbomer | 0,15 |
| | Wasser | 4,85 |
| E | | |
| | Sodium hydroxide | 0,90 |
| F | | |
| | Phenoxyethanol, butylparaben, ethylparaben, propylparaben, methylparaben | 0,50 |
| | Parfum | 0,30 |

**Herstellung:**

Phasen A und B getrennt voneinander auf 80 °C erhitzen. Phase B mit dem Thurrax homogenisieren, bis das Pigment schön benetzt ist. Phase B zu Phase A geben und 2 Min. homogenisieren. Auf 35 °C abkühlen, Phase C zufügen und 30 Sek. homogenisieren. Phase D zufügen und 30 Sek. homogenisieren. Phase E unterrühren und mit Phase F neutralisieren und homogenisieren bis eine zufriedenstellende Pigmentverteilung erreicht ist (mikroskopische Kontrolle!). Auf Raumtemperatur abkühlen, entlüften und Phase G einrühren.

**Rezepturbeispiel 10: Sun Protection Lotion (PEG-free) in vitro SPF (Diffey) 12 ± 2**

| Rohstoff (INCI) | % |
|---|---|
| A | |
| C12-15 Alkyl benzoate | 3,00 |
| Decyl cocoate | 4,00 |
| Ethylhexylpalmitate | 3,00 |
| Glyceryl stearate | 0,50 |
| Stearic acid | 0,50 |
| Tocopheryl acetate | 0,50 |
| B | |
| Cetearyl glucoside | 1,50 |
| Propylene glycol | 2,00 |
| Glycerin | 1,00 |
| Wasser | 76,80 |
| C | |
| Produkt aus Beispiel 2a | 5,00 |
| D | |
| Carbomer | 0,20 |
| Paraffinum liquidum (mineral oli) | 0,80 |
| E | |
| Sodium hydroxide | 0,50 |
| F | |
| Propylene glycol, diazolidinyl urea, methylparaben, propylparaben | 0,50 |
| Parfum | 0,20 |

**Herstellung:**

Phase A und Phase B getrennt auf 80 °C erhitzen. Phase A unter Rühren zu Phase B geben. Bei 40 °C Phase C unter Rühren in die Emulsion eintragen und homogenisieren bis zur optimalen Pigmentverteilung. Bei 35 °C Phase D zugeben und nochmals kurz homogenisieren. Phase E zugeben, pH-Wert kontrollieren und nochmals kurz homogenisieren. Phase F zugeben und kalt rühren.

**Rezepturbeispiel 11: W/O Sonnenschutzlotion mit anorganischem Filter, in vitro SPF (Diffey-Methode) 8,7 ± 1,6, UVA-PF 4,4 ± 0,5**

| Rohstoff (INCI) | % |
|---|---|
| A | |
| Cetyl PEG/PPG-10/1 Dimethicone | 2,50 |
| Stearoxy dimethicone | 0,25 |
| Ethylhexyl stearate | 12,75 |
| Ethylhexyl palmitate | 8,00 |
| Isohexadecane | 7,00 |
| hydrogenate castor oil | 0,50 |
| Ceresin (microcrystalline wax) | 1,00 |
| B | |
| Produkt aus Beispiel 2b | 5,00 |
| C | |
| Wasser | 62,00 |
| Sodium chloride | 0,50 |
| Propylene glycol, diazolidinyl usrea, methylparaben, propylparaben | 0,50 |

**Herstellung:**

Phase A auf 80 °C erhitzen. Das Titandioxid (Phase B) sorgfältig in die heiße Ölphase einarbeiten. Phase C langsam unter Rühren (500 upm, (Mig-Rührer) zu Phase A/B geben. Bei 1600 upm für 2 Minuten homogenisieren. Unter Rühren ca. 300 upm) auf ca. 40 °C abkühlen und nochmals für 2 Minuten bei 1600 upm homogenisieren.

**Rezepturbeispiel 12**

Im Folgenden werden beispielhaft Rezepturen für kosmetische Zubereitungen angegeben, die in gleicher Weise mit Titandioxid nach Beispiel 2a, 2b, 2c oder 2d enthalten werden (In der Tabelle jewels als Titanium dioxide bezeichnet). Im übrigen sind die INCI-Bezeichnungen der handelsüblichen Verbindungen angegeben.

UV-Pearl, OMC steht für die Zubereitung mit der INCI-Bezeichnung: Water (for EU: Aqua), Ethylhexyl Methoxycinnamate, Silica, PVP, Chlorphenesin, BHT; diese Zubereitung ist im handel unter der Bezeichnung Eusolex®UV Pearl™MOMC von der Merck KGaA, Darmstadt erhältlich.
Die anderen in den Tabellen angegebenen UV-Pearl sind jeweils analog zusammengesetzt, wobei OMC gegen die angegebenen UV-Filter ausgetauscht wurde.

Tabelle 1 W/O-Emulsionen (Zahlen in Gew.-%)

| | 1-1 | 1-2 | 1-3 | 1-4 | 1-5 | 1-6 | 1-7 | 1-8 | 1-9 | 1-10 |
|---|---|---|---|---|---|---|---|---|---|---|
| Titanium dioxide | 3 | 2 | 5 | 10 | 7 | 4 | 15 | 1 | 3 | 3 |
| Butylmethoxydibenzoylmethane | 5 | 3 | 2 | 1 | 2 | | | | 1 | 1 |
| Zinc oxide | | | | | | | | 5 | 2 | |
| UV-Pearl , OMC | 30 | 15 | 15 | 15 | 15 | 15 | 15 | 15 | 15 | 15 |
| Polyglyceryl-3-Dimerate | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 |
| Cyclomethicone | 0,5 | 0,5 | 0,5 | 0,5 | 0,5 | 0,5 | 0,5 | 0,5 | 0,5 | 0,5 |
| Propylparabene | 0,0 5 | 0,0 5 | 0,0 5 | 0,0 5 | 0,0 5 | 0,0 5 | 0,0 5 | 0,0 5 | 0,0 5 | 0,0 5 |
| Methylparabene | 0,1 5 | 0,1 5 | 0,1 5 | 0,1 5 | 0,1 5 | 0,1 5 | 0,1 5 | 0,1 5 | 0,1 5 | 0,1 5 |
| Water | ad 100 | ad 100 | ad 100 | ad 100 | ad 100 | ad 100 | ad 100 | ad 100 | ad 100 | ad 100 |
| | 1-11 | 1-12 | 1-13 | 1-14 | 1-15 | 1-16 | 1-17 | 1-18 | | |
| Titanium dioxide | 3 | 5 | 2 | 4 | 3 | 1 | 2 | 5 | | |
| Benzylidene malonate polysiloxane | | 1 | 0,5 | | | | | | | |
| Methylene Bis-Benztriazolyl Tetramethylbutylphenol | 1 | 1 | 0,5 | | | | | | | |
| Dihydroxyavcetone | 5 | 3 | 2 | 5 | 1 | 3 | 7 | 2 | | |
| Polyglyceryl-3-Dimerate | 3 | 3 | 3 | 3 | | | | | | |

(fortgesetzt)

| | 1-11 | 1-12 | 1-13 | 1-14 | 1-15 | 1-16 | 1-17 | 1-18 |
|---|---|---|---|---|---|---|---|---|
| Cera Alba | 0,3 | 0,3 | 0,3 | 0,3 | 2 | 2 | 2 | 2 |
| Hydrogenated Castor Oil | 0,2 | 0,2 | 0,2 | 0,2 | | | | |
| Paraffinium Liquidum | 7 | 7 | 7 | 7 | | | | |
| Caprylic/Capric Triglyceride | 7 | 7 | 7 | 7 | | | | |
| Hexyl Laurate | 4 | 4 | 4 | 4 | | | | |
| PVP/Eicosene Copolymer | 2 | 2 | 2 | 2 | | | | |
| Propylene Glycol | 4 | 4 | 4 | 4 | | | | |
| Magnesium Sulfate | 0,6 | 0,6 | 0,6 | 0,6 | | | | |
| Tocopherol | 0,5 | 0,5 | 0,5 | 0,5 | | | | |
| Tocopheryl Acetate | 0,5 | 0,5 | 0,5 | 0,5 | 1 | 1 | 1 | 1 |
| Cyclomethicone | 0,5 | 0,5 | 0,5 | 0,5 | | | | |
| Propylparabene | 0,0 5 | 0,0 5 | 0,0 5 | 0,0 5 | 0,0 5 | 0,0 5 | 0,0 5 | 0,0 5 |
| Methylparabene | 0,1 5 | 0,1 5 | 0,1 5 | 0,1 5 | 0,1 5 | ob 5 | 0,1 5 | 0,1 5 |
| Dicocoyl Pentyerythrityl Citrate (and) Sorbitan Sesquioleate (and) Cera Alba (and) Aluminium Stearate | | | | | 6 | 6 | 6 | 6 |
| PEG-7 Hydrogenated Castor Oil | | | | | 1 | 1 | 1 | 1 |
| Zinc Stearate | | | | | 2 | 2 | 2 | 2 |
| Oleyl Erucate | | | | | 6 | 6 | 6 | 6 |
| Decyl Oleate | | | | | 6 | 6 | 6 | 6 |
| Dimethicone | | | | | 5 | 5 | 5 | 5 |
| Tromethamine | | | | | 1 | 1 | 1 | 1 |
| Glycerin | | | | | 5 | 5 | 5 | 5 |
| Allantoin | | | | | 0,2 | 0,2 | 0,2 | 0,2 |
| water | ad 100 | ad 100 | ad 100 | ad 100 | ad 100 | ad 100 | ad 100 | ad 100 |

(fortgesetzt)

| | 1-19 | 1-20 | 1-21 | 1-22 | 1-23 | 1-24 | 1-25 | 1-26 | 1-27 | 1-28 | 1-29 |
|---|---|---|---|---|---|---|---|---|---|---|---|
| Titanium dioxide | 1 | 2 | 5 | 1 | 3 | 4 | 5 | 2 | 3 | 3 | 3 |
| Benzylidene malonate polysiloxane | | | | 1 | | | | | 1 | 1 | |
| Methylene Bis-Benztriazolyl Tetramethylbutylphenol | | | | | | 1 | 2 | 1 | | | 1 |
| Zinc oxide | | | | | | | | 5 | 2 | | |
| UV-Pearl OMC | 5 | 5 | 5 | 5 | 7 | 5 | 5 | 5 | 5 | 5 | 8 |
| UV-Pearl , OCR | | 10 | | | | | | | | | 5 |
| UV-Pearl, EthylhexylDimethylPABA | | | 10 | | | | | | | | |
| UV-Pearl, Homosalate | | | | 10 | | | | | | | |
| UV-Pearl, Ethylhexyl salicylate | | | | | 10 | | | | | | |
| UV-Paarl, OMC, BP-3 | | | | | | 10 | | | | | |
| UV-Pearl, OCR, BP-3 | | | | | | | 10 | | | | |
| UV-Pearl, Ethylhexyl Dimethyl PABA, BP-3 | | | | | | | | 10 | | | |
| UV-Pearl, Homosalate, BP-3 | | | | | | | | | 10 | | |
| UV-Pearl, Ethylhexyl salicylate, BP-3 | | | | | | | | | | 10 | |
| Butylmethoxydibenzoylmethane | | | | | | | | | | | 2 |
| UV-Pearl OMC, 4-Methylbenzylidene Camphor | 25 | | | | | | | | | | |
| Polyglyceryl-3-Dimerate | 3 | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 | 0,6 |

(fortgesetzt)

| | 1-19 | 1-20 | 1-21 | 1-22 | 1-23 | 1-24 | 1-25 | 1-26 | 1-27 | 1-28 | 1-29 |
|---|---|---|---|---|---|---|---|---|---|---|---|
| Tocopherol | 0,5 | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 | 0,5 |
| Cyclomethicone | 0,5 | 0,5 | 0,5 | 0,5 | 0,5 | 0,5 | 0,5 | 0,5 | 0,5 | 0,5 | 0,5 |
| Propylparabene | 0,0 5 | 0,0 5 | 0,0 5 | 0,0 5 | 0,0 5 | 0,0 5 | 0,0 5 | 0,0 5 | 0,0 5 | 0,0 5 | 0,0 5 |
| Methylparabene | 0,1 5 | 0,1 5 | 0,1 5 | 0,1 5 | 0,1 5 | 0,1 5 | 0,1 5 | 0,1 5 | 0,1 5 | 0,1 5 | 0,1 5 |
| Water | ad 100 | | | | | | | | | | |

Tabelle 2: O/W-Emulsionen, Zahlen in Gew.-%

| | 2-1 | 2-2 | 2-3 | 2-4 | 2-5 | 2-6 | 2-7 | 2-8 | 2-9 | 2-10 |
|---|---|---|---|---|---|---|---|---|---|---|
| Titanium dioxide | 3 | 2 | 5 | 2 | 5 | 2 | 5 | 2 | 5 | 3 |
| Methylene Bis-Benztriazolyl Tetramethylbutylphenol | | | | | | 1 | 2 | 1 | | |
| Butylmethoxydibenzoylmetha ne | 3 | 3 | 3 | 3 | 3 | 3 | 3 | 3 | 3 | 3 |
| 2-(4-Hydroxy-3,5-dimethoxy-benzyliden)-malonsäure-bis-(2-ethyl-hexyl)ester | 1 | 5 | 4 | | 6 | | 7 | | 2 | 1 |
| 4-Methylbenzyliden Camphor | 2 | 2 3 | 3 | | 4 | | 3 | | 2 | |
| Stearyl Alcohol (and) Steareth-7 (and) Steareth-10 | 3 | 3 | 3 | 3 | 3 | 3 | 3 | 3 | 3 | 3 |
| Glyceryl Stearate (and) Ceteth-20 | 3 | 3 | 3 | 3 | 3 | 3 | 3 | 3 | 3 | 3 |
| Microwax | 1 | 1 | 1 | 1 | 1 | 1 | 1 | 1 | 1 | 1 |
| Cetearyl Octanoate | 11, 5 | 11, 5 | 11, 5 | 11, 5 | 11, 5 | 11, 5 | 11, 5 | 11, 5 | 11, 5 | 11, 5 |
| Caprylic/Capric Triglyceride | 6 | 6 | 6 | 6 | 6 | 6 | 6 | 6 | 6 | 6 |
| Oleyl Oleate | 6 | 6 | 6 | 6 | 6 | 6 | 6 | 6 | 6 | 6 |
| Propylene Glycol | 4 | 4 | 4 | 4 | 4 | 4 | 4 | 4 | 4 | 4 |
| Propylparabene | 0,0 5 | 0,0 5 | 0,0 5 | 0,0 5 | 0,0 5 | 0,0 5 | 0,0 5 | 0,0 5 | 0,0 5 | 0,0 5 |
| Methylparabene | 0,1 5 | 0,1 5 | 0,1 5 | 0,1 5 | 0,1 5 | 0,1 5 | 0,1 5 | 0,1 5 | 0,1 5 | 0,1 5 |
| Tromethamine | | | 1,8 | | | | | | | |
| Water | ad 100 | ad 100 | ad 100 | Ad 100 | ad 100 | ad 100 | ad 100 | ad 100 | ad 100 | ad 100 |
| | 2-11 | 2-12 | 2-13 | 2-14 | 2-15 | 2-16 | 2-17 | 2-18 | | |
| Titanium dioxide | 3 | 1 | 2 | 5 | 4 | 3 | 2 | 5 | | |
| Benzylidene malonate polysiloxane | | 1 | 0,5 | | | | | | | |
| Methylene Bis-Benztriazolyl Tetramethylbutylphenol | 1 | 1 | 0,5 | | | | | | | |
| Butylmethoxydibenzoylmethane | 3 | 3 | 3 | 3 | 3 | 3 | 3 | 3 | | |
| 2-(4-Hydroxy-3,5-dimethoxy-benzyliden)-malonsäure-bis-(2-ethyl-hexyl)ester | 1 | 5 | 4 | | 6 | | 7 | | | |
| Zinc oxide | | | 2 | | | | | | | |
| UV-Pearl , OMC | 15 | 15 | 15 | 30 | 30 | 30 | 15 | 15 | | |

(fortgesetzt)

| | 2-11 | 2-12 | 2-13 | 2-14 | 2-15 | 2-16 | 2-17 | 2-18 | | |
|---|---|---|---|---|---|---|---|---|---|---|
| 4-Methylbenzyliden Camphor | | | | 3 | | | | | | |
| Phenylbenzimidazole Sulfonic Acid | | | | | 4 | | | | | |
| Stearyl Alcohol (and) Steareth-7 (and) Steareth-10 | 3 | 3 | 3 | 3 | | | | | | |
| Glyceryl Stearate | 3 | 3 | 3 | 3 | | | | | | |
| Microwax | 1 | 1 | 1 | 1 | | | | | | |
| Cetearyl Octanoate | 11,5 | 11,5 | 11,5 | 11,5 | | | | | | |
| Caprylic/Capric Triglyceride | 6 | 6 | 6 | 6 | 14 | 14 | 14 | 14 | | |
| Oleyl Oleate | 6 | 6 | 6 | 6 | | | | | | |
| Propylene Glycol | 4 | 4 | 4 | 4 | | | | | | |
| Glyceryl Stearate SE | | | | | 6 | 6 | 6 | 6 | | |
| Stearic Acid | | | | | 2 | 2 | 2 | 2 | | |
| Persea Gratissima | | | | | 8 | 8 | 8 | 8 | | |
| Propylparabene | 0,05 | 0,05 | 0,05 | 0,05 | 0,05 | 0,05 | 0,05 | 0,05 | | |
| Methylparabene | 0,15 | 0,15 | 0,15 | 0,15 | 0,15 | 0,15 | 0,15 | 0,15 | | |
| Tromethamine | | | | 1,8 | 1,8 | | | | | |
| Glycerin | | | | | 3 | 3 | 3 | 3 | | |
| Water | ad 100 | ad 100 | Ad 100 | ad 100 | ad 100 | ad 100 | ad 100 | ad 100 | | |
| | 2-19 | 2-20 | 2-21 | 2-22 | 2-23 | 2-24 | 2-25 | 2-26 | 2-27 | 2-28 |
| Titanium dioxide | 10 | 5 | 7 | 8 | 2 | 1 | 3 | 3 | 6 | 2 |
| Benzylidene malonate polysiloxane | 1 | 2 | | | | 1 | 1 | | 1 | 0,5 |
| Butylmethoxydibenzoylmethane | 3 | 3 | 3 | 3 | 3 | 3 | 3 | 3 | 3 | 3 |
| Octocrylene | 1 | 5 | 4 | | 6 | | 7 | | 2 | 1 |
| Methylene Bis-Benztriazolyl Tetramethylbutylphenol | | | 1 | 2 | 1 | | | 1 | 1 | 0,5 |
| Zinc oxide | | | | | 5 | 2 | | | | 2 |
| UV-Pearl, OMC | 15 | 15 | 15 | 15 | 15 | 15 | 15 | 15 | 15 | 15 |

(fortgesetzt)

| | 2-19 | 2-20 | 2-21 | 2-22 | 2-23 | 2-24 | 2-25 | 2-26 | 2-27 | 2-28 |
|---|---|---|---|---|---|---|---|---|---|---|
| Caprylic/Capric Triglyceride | 14 | 14 | 14 | 14 | 14 | 14 | 14 | 14 | 14 | 14 |
| Glyceryl Stearate SE | 6 | 6 | 6 | 6 | 6 | 6 | 6 | 6 | 6 | 6 |
| Stearic Acid | 2 | 2 | 2 | 2 | 2 | 2 | 2 | 2 | 2 | 2 |
| Persea Gratissima | 8 | 8 | 8 | 8 | 8 | 8 | 8 | 8 | 8 | 8 |
| Propylparabene | 0,0 5 | 0,0 5 | 0,0 5 | 0,0 5 | 0,0 5 | 0,0 5 | 0,0 5 | 0,0 5 | 0,0 5 | 0,0 5 |
| Methylparabene | 0,1 5 | 0,1 5 | 0,1 5 | 0,1 5 | 0,1 5 | 0,1 5 | 0,1 5 | 0,1 5 | 0,1 5 | 0,1 5 |
| Glycerin | 3 | 3 | 3 | 3 | 3 | 3 | 3 | 3 | 3 | 3 |
| Water | ad 100 | ad 100 | ad 100 | Ad 100 | ad 100 | ad 100 | ad 100 | ad 100 | ad 100 | ad 100 |

Tabelle 3: Gele, Zahlen in Gew.-%

| | 3-1 | 3-2 | 3-3 | 3-4 | 3-5 | 3-6 | 3-7 | 3-8 | 3-9 | 3-10 |
|---|---|---|---|---|---|---|---|---|---|---|
| a = aqueaous gel | | | | | | | | | | |
| Titanium dioxide | 5 | 2 | 5 | 1 | 1 | 1 | 1 | 1 | 3 | 3 |
| Butylmethoxydibenzoylmethane | 2 | 2 | 2 | 2 | 2 | 2 | 2 | 2 | 2 | 2 |
| Dihydroxyacetone | 1 | 5 | 4 | | 6 | | 7 | | 2 | 1 |
| Benzylidene malonate polysiloxane | | | 1 | 1 | 2 | | | | 1 | 1 |
| Methylene Bis-Benztriazolyl Tetramethylbutylphenol | | 1 | | | | 1 | 2 | 1 | | |
| Zinc oxide | | | | 2 | | | | 5 | 2 | |
| UV-Pearl Ethylhexyl Mehtoxycinnamat | 30 | 15 | 15 | 15 | 15 | 15 | 15 | 15 | 15 | 15 |
| 4-Methylbenzyliden Camphor | | | | | 2 | | | | | |
| Prunus Dulcis | 5 | 5 | 5 | 5 | 5 | 5 | 5 | 5 | 5 | 5 |
| Tocopheryl Acetate | 0,5 | 0,5 | 0,5 | 0,5 | 0,5 | 0,5 | 0,5 | 0,5 | 0,5 | 0,5 |
| Caprylic/Capric Triglyceride | 3 | 3 | 3 | 3 | 3 | 3 | 3 | 3 | 3 | 3 |
| Octyldodecanol | 2 | 2 | 2 | 2 | 2 | 2 | 2 | 2 | 2 | 2 |
| Decyl Oleate | 2 | 2 | 2 | 2 | 2 | 2 | 2 | 2 | 2 | 2 |
| PEG-8 (and) Tocopherol (and) Ascorbyl Palmitate (and) Ascorbic Acid (and) Citric Acid | 0,0 5 | 0,0 5 | 0,0 5 | 0,0 5 | 0,0 5 | 0,0 5 | 0,0 5 | 0,0 5 | 0,0 5 | 0,0 5 |
| Sorbitol | 4 | 4 | 4 | 4 | 4 | 4 | 4 | 4 | 4 | 4 |
| Polyacrylamide (and) C13-14 Isoparaffin (and) Laureth-7 | 3 | 3 | 3 | 3 | 3 | 3 | 3 | 3 | 3 | 3 |
| Propylparabene | 0,0 5 | 0,0 5 | 0,0 5 | 0,0 5 | 0,0 5 | 0,0 5 | 0,0 5 | 0,0 5 | 0,0 5 | 0,0 5 |
| Methylparabene | 0,1 5 | 0,1 5 | 0,1 5 | 0,1 5 | 0,1 5 | 0,1 5 | 0,1 5 | 0,1 5 | 0,1 5 | 0,1 5 |
| Tromethamine | | | 1,8 | | | | | | | |
| Water | ad 100 | ad 100 | ad 100 | ad 100 | ad 100 | ad 100 | ad 100 | ad 100 | ad 100 | ad 100 |
| | 3-11 | 3-12 | 3-13 | | | | | | | |
| a = aqueaous gel | | | | | | | | | | |
| Titanium dioxide | 3 | 1 | 2 | | | | | | | |
| Benzylidene malonate polysiloxane | | 1 | 0,5 | | | | | | | |

(fortgesetzt)

| | 3-11 | 3-12 | 3-13 | | | | | |
|---|---|---|---|---|---|---|---|---|
| Methylene Bis-Benztriazolyl Tetramethylbutylphenol | 1 | 1 | 0,5 | | | | | |
| Butylmethoxydibenzoylmethane | 2 | 2 | 2 | | | | | |
| 2-(4-Hydroxy-3,5-dimethoxy-benzyliden)-malonsäure-bis-(2-ethyl-hexyl)ester | 1 | 5 | 4 | | | | | |
| Zinc oxide | | | 2 | | | | | |
| UV-Pearl Ethylhexyl Mehtoxycinnamat | 15 | 15 | 15 | | | | | |
| Prunus Dulcis | 5 | 5 | 5 | | | | | |
| Tocopheryl Acetate | 0,5 | 0,5 | 0,5 | | | | | |
| Caprylic/Capric Triglyceride | 3 | 3 | 3 | | | | | |
| Octyldodecanol | 2 | 2 | 2 | | | | | |
| Decyl Oleate | 2 | 2 | 2 | | | | | |
| PEG-8 (and) Tocopherol (and) Ascorbyl Palmitate (and) Ascorbic Acid (and) Citric Acid | 0,0 5 | 0,0 5 | 0,0 5 | | | | | |
| Sorbitol | 4 | 4 | 4 | | | | | |
| Polyacrylamide (and) C13-14 Isoparaffin (and) Laureth-7 | 3 | 3 | 3 | | | | | |
| Carbomer | | | | | | | | |
| Propylparabene | 0,0 5 | 0,0 5 | 0,0 5 | | | | | |
| Methylparabene | 0,1 5 | 0,1 5 | 0,1 5 | | | | | |
| Allantoin | | | | | | | | |
| Tromethamine | | | | | | | | |
| Water | ad 100 | ad 100 | Ad 100 | | | | | |
| | 3-14 | 3-15 | 3-16 | 3-17 | 3-18 | 3-19 | 3-20 | 3-21 |
| Titanium dioxide | 1 | 5 | 3 | 1 | 2 | 8 | 12 | 1 |
| Butylmethoxydibenzoylmethane | 2 | 2 | 2 | 2 | 2 | 2 | 2 | 2 |
| UV-Pearl, OMC | 15 | 10 | | 10 | 10 | 10 | 15 | 10 |
| UV-Pearl , OCR | | | 10 | | | | | |

(fortgesetzt)

| | 3-14 | 3-15 | 3-16 | 3-17 | 3-18 | 3-19 | 3-20 | 3-21 |
|---|---|---|---|---|---|---|---|---|
| UV-Pearl , OMC, Methylene Bis-Benztriazolyl Tetramethylbutylphenol | | 7 | | 6 | | | | |
| UV-Pearl, Ethylhexyl salicylate, Butylmethoxydibenzoylmethane | | | 10 | | | | | |
| Disodium Phenyl Dibenzimidazole Tetrasulfonate | | 3 | | | | 3 | | 3 |
| Phenylbenzimidazole Sulfonic Acid | | 2 | | | 2 | 3 | | 3 |
| Prunus Dulcis | 5 | 5 | 5 | | | | | |
| Tocopheryl Acetate | 0,5 | 0,5 | 0,5 | | | | | |
| Caprylic/Capric Triglyceride | 3 | 3 | 3 | | | | | |
| Octyldodecanol | 2 | 2 | 2 | | | | | |
| Decyl Oleate | 2 | 2 | 2 | | | | | |
| PEG-8 (and) Tocopherol (and) Ascorbyl Palmitate (and) Ascorbic Acid (and) Citric Acid | 0,0 5 | 0,0 5 | 0,0 5 | | | | | |
| Sorbitol | 4 | 4 | 4 | 5 | 5 | 5 | 5 | 5 |
| Polyacrylamide (and) C13-14 Isoparaffin (and) Laureth-7 | 3 | 3 | 3 | | | | | |
| Carbomer | | | | 1,5 | 1,5 | 1,5 | 1,5 | 1,5 |
| Propylparabene | 0,0 5 | 0,0 5 | 0,0 5 | | | | | |
| Methylparabene | 0,1 5 | 0,1 5 | 0,1 5 | 0,1 5 | 0,1 5 | 0,1 5 | 0,1 5 | 0,1 5 |
| Allantoin | | | | 0,2 | 0,2 | 0,2 | 0,2 | 0,2 |
| Tromethamine | | | | 2,4 | 2,4 | 2,4 | 2,4 | 2,4 |
| Water | Ad 100 | Ad 100 | ad 100 | ad 100 | ad 100 | ad 100 | ad 100 | ad 100 |

**Rezepturbeispiel 13: Sonnenschutzspray**

| | | |
|---|---|---|
| A) | CERALUTION® C; Fa. Sasol | 15.0% |
| B) | Produkt gemäß Beispiel 2a | 5.0% |
| | Ethylhexyl Methoxycinnamate | 4.8% |
| | Ethylhexyl Salicylate | 4.8% |
| | Tocopheryl Acetate | 0.6% |
| | Cyclomethicone | 1.0% |
| | C12-15 Alkyl Benzoate | 2.5% |
| | Tridecyl Salicylate | 2.5% |
| C) | Water (Aqua), Deionized | 38.3% |
| | Water (Aqua), Deionized with 4% Avicel CL 611 (Microcrystalline Cellulose (and) Cellulose Gum) | 25.0% |
| D) | Phenoxyethanol (and) Methylparaben (and) Ethylparaben (and) Butylparaben (and) Propylparaben (and) Isobutylparaben) | 0.5% |
| E) | Fragrance q.s. | |

Herstellung: Phase B wird bei Raumtemperatur unter rühren langsam zu Phase A gegeben. Danach wird Phase C zugegeben. Anschließend werden die Phasen D und E zugegeben.

INCI CERALUTION® C:

Aqua (and) Capric / Caprylic triglyceride (and) Glycerin (and) Ceteareth-25 (and) Sodium Dicocoylethylenediamine PEG-15 Sulfate (and) Sodium Lauroyl Lactylate (and) Behenyl Alcohol (and) Glyceryl Stearate (and) Glyceryl Stearate Citrate (and) Gum Arabic (and) Xanthan Gum (and) Phenoxyethanol (and) Methylparaben (and) Ethylparaben (and) Butylparaben (and) Isobutylparaben

**Rezepturbeispiel 14: Sonnenschutzlotion (O/W); SPF 7,6 (Sun Protection Factor, Diffey Methode)**

| | % |
|---|---|
| **A** | |
| Stearyl Alcohol (and) Steareth-7 (and) Steareth-10 | 3,00 |
| Glyceryl Stearate (and) Ceteth-20 | 3,00 |
| Cetearyl Octanoate | 15,50 |
| Glyceryl Stearate | 3,00 |
| Oleyl Oleate | 7,00 |
| Microwax | 1,00 |
| Caprylic/Capric Triglyceride | 6,00 |
| **B** | |
| Produkt nach Beispiel 2c | 5,00 |
| Propylene Glycol | 4,00 |
| Konservierungsmittel | q. s. |
| Wasser, demineralisiert | ad 100,00 |

**<u>Herstellung:</u>**

Titandioxid in Phase B einrühren und auf 80 °C erhitzen. Phase A auf 75 °C erhitzen. Phase B unter Rühren

langsam zu Phase A geben, homogenisieren und unter Rühren abkühlen.

**Rezepturbeispiel 15: Sonnencreme ohne organ. Filter (W/O); In vitro SPF (Diffey) 32 +/- 5**

| **Rohstoff** | **INCI** | **[%]** |
|---|---|---|
| A | | |
| Wasser, demineralisiert | AQUA (WATER) | 53,40 |
| Polyethylenglycol 400 | PEG-8 | 4,00 |
| Pemulen TR-1 | ACRYLATES/C10-30 ALKYL ACRYLATE CROSSPOLYMER | 0,20 |
| Natronlauge, 10 %ig | SODIUM HYDROXIDE | 0,90 |
| STEPAN-MILD RM-1 | SODIUM STEARYL PHTHALAMATE | 1,00 |
| B1 | | |
| Ceraphyl 368 | ETHYLHEXYL PALMITATE | 10,00 |
| Zink Oxid | ZINC OXIDE | 3,00 |
| Imwitor 900 | GLYCERYL STEARATE | 0,50 |
| Jojobaöl | BUXUS CHINENSIS (JOJOBA OIL) | 1,00 |
| B2 | | |
| Germaben II | PROPYLENE GLYCOL, DIAZOLIDINYL, UREA, METHYLPARABEN, PROPYLPARABEN | 1,00 |
| Tegosoft TN | C12-15 ALKYL BENZOATE | 15,00 |
| Antaron V-216 | PVP/HEXADECENE COPOLYMER | 2,00 |
| Produkt nach Beispiel 2d | | 8,00 |

**Herstellung:**

1. Wasser in Gefäß mit Heizmöglichkeit und Rührer vorlegen (z.B. Eurostar digital mixer, IKA).
2. PEG-400 zugeben, dann Pemulen TR-1 unter rühren in die Wasserphase eintragen, bis es homogen verteilt ist.
3. Natronlauge zugeben um das Pemulen TR-1 zu aktivieren, rühren bis ein klares Gel vorliegt.
4. Aufheizen der Wasserphase bis auf 72-75°C.
5. Stepan-Mild RM1 be 70°C mit geringer Rührgeschwindigkeit eintragen und auf 70-72°C aufheiten. Mindestens 15 Minuten bei dieser Temperatur rühren bis das Stepanmild RM1 gut verteilt ist.
6. Ölphase in einem separaten Gefäß herstellen und auf 75°C aufheizen. Bei 60°C Imwitor 900 und Jojoba Öl zugeben. Weiter aufheizen und bei 75°C Ölphase B zur Wasserphase mit erhöhter Rührgeschwindigkeit geben und 10 Minuten weiter rühren.
7. Ölphase B2 in einem weiteren Gefäß herstellen. Tegosoft TN und Antaron V-216 auf 85°C aufheizen. Bei 75°C Titandioxid zugeben und für 20 Minuten dispergieren bis gute Pigmentverteilung erreicht ist evtl. homogenisieren. Ölphase B2 zur Emulsion aus Punkt 6 geben und für 20-25 Minuten bei 72-75°C weiter emulgieren.
9. Kühlung mit moderater Rührleistung beginnen
10. Germaben II beio < 40°C unter rühren zugeben.
11. Bei t<35°C am U-Turax für 5 Minuten bei 5000 upm homogenisieren..
13. Auf Raumtemperatur abkühlen und entlüften
14. Über Nacht ruhen und am nächsten Tag abfüllen

**Beschreibung der Abbildungen**

**Figur 1:** Transmissionselektronenmikroskopische Aufnahme von Titandioxid-Kristalliten hergestellt gemäß Beispiel 1 a.

**Figur 2:** Transmissionselektronenmikroskopische Aufnahme von Titandioxid-Kristalliten hergestellt gemäß Beispiel 2a.

**Figur 3:** Gehalt von 4,4'-Methoxy-tert.-butyldibenzoylmethan (BMDBM) in Formulierungen mit Titandioxid in Abhängigkeit von den Lagerbedingungen gemäß Beispiel 3; Startkonzentration 3% BMDBM; (Beispiel 3a: erfindungsgemäßes Beispiel; Beispiel 3b: Vergleichsbeispiel)

Messpunkte:

A: unmittelbar nach Herstellung der Formulierung
B: 4 Wochen Lagerung bei Raumtemperatur im Dunkeln
C: 4 Wochen Lagerung bei 5°C im Dunkeln
D: 4 Wochen Lagerung bei 40°C im Dunkeln
E: 12 Wochen Lagerung bei Raumtemperatur im Dunkeln
F: 12 Wochen Lagerung bei 5°C im Dunkeln
G: 12 Wochen Lagerung bei 40°C im Dunkeln

**Figur 4:** b*-Werte (L*a*b*-System; CIELab, DIN 6174) kosmetischer Formulierungen nach 3 Monaten Lagerung bei 50°C im Dunkeln gemäß Beispiel 6.

## Patentansprüche

1. Verfahren zur Herstellung eines nanopartikulären UV-Schutzmittels, **dadurch gekennzeichnet, dass**

   a) ein nanopartikuläres Metalloxid hydrothermal behandelt wird, in dem die Hydrothermalbehandlung in einem geschlossenen Behälter bei Temperaturen im Bereich von 140 bis 360°C, durchgeführt wird
   und
   b) anschließend eine Siliciumdioxid-Beschichtung aufgebracht wird.

2. Verfahren nach Anspruch 1, **dadurch gekennzeichnet, dass** in Schritt a) ein nanopartikuläres Titandioxid hydrothermal behandelt wird.

3. Verfahren nach Anspruch 1 oder 2, **dadurch gekennzeichnet, dass** Schritt b) als Sol-Gel-Prozess durchgeführt wird, wobei vorzugsweise eine Wasserglaslösung zu einer Suspension des Metalloxids gegeben wird.

4. Verfahren nach einem oder mehreren der Ansprüche 1 bis 3, **dadurch gekennzeichnet, dass** Schritt b) bei einem im Bereich von pH = 2 bis pH = 11 konstant gehaltenen pH-Wert erfolgt.

5. Verfahren nach einem oder mehreren der Ansprüche 1 bis 4, **dadurch gekennzeichnet, dass** Schritt b) nach vorheriger pH-Einstellung der Suspension des Metalloxides auf einen Wert von pH = 7 bis pH = 11 ohne pH-Regelung erfolgt und der pH-Wert anschließend auf einen pH = 5 bis pH = 8 abgesenkt wird.

6. Verfahren nach einem oder mehreren der Ansprüche 1 bis 5, **dadurch gekennzeichnet, dass** Schritt b) bei erhöhter Temperatur, vorzugsweise bei einer Temperatur im Bereich von 50°C bis 100°C, durchgeführt wird.

## Claims

1. Process for the preparation of a nanoparticulate UV protectant, **characterised in that**

   a) a nanoparticulate metal oxide is subjected to hydrothermal treatment, where the hydrothermal treatment is carried out in a closed container at temperatures in the range from 140 to 360°C
   and
   b) a silicon dioxide coating is subsequently applied.

2. Process according to Claim 1, **characterised in that** in step a) a nanoparticulate titanium dioxide is subjected to hydrothermal treatment.

3. Process according to Claim 1 or 2, **characterised in that** step b) is carried out as a sol-gel process, in which a water-glass solution is preferably added to a suspension of the metal oxide.

4. Process according to one or more of Claims 1 to 3, **characterised in that** step b) is carried out at a pH kept constant in the range from pH = 2 to pH = 11.

5. Process according to one or more of Claims 1 to 4, **characterised in that** step b) is carried out without pH regulation after prior adjustment of the pH of the suspension of the metal oxide to a value of pH = 7 to pH = 11, and the pH is subsequently lowered to a pH = 5 to pH = 8.

6. Process according to one or more of Claims 1 to 5, **characterised in that** step b) is carried out at elevated temperature, preferably at a temperature in the range from 50°C to 100°C.

## Revendications

1. Procédé de préparation d'un protecteur anti-UV nanoparticulaire, **caractérisé en ce que**

   a) un oxyde de métal nanoparticulaire est soumis à un traitement hydrothermal, où le traitement hydrothermal est effectué dans un conteneur fermé à des températures dans la plage allant de 140 à 360°C
   et
   b) un revêtement à base de dioxyde de silicium est ensuite appliqué.

2. Procédé selon la revendication 1, **caractérisé en ce que**, dans l'étape a), un dioxyde de titane nanoparticulaire est soumis à un traitement hydrothermal.

3. Procédé selon la revendication 1 ou 2, **caractérisé en ce que** l'étape b) est effectuée sous forme de procédé sol-gel, où une solution de verre soluble est ajoutée de préférence à une suspension de l'oxyde de métal.

4. Procédé selon l'une ou plusieurs parmi les revendications 1 à 3, **caractérisé en ce que** l'étape b) est effectuée à un pH maintenu constant dans la plage allant de pH = 2 à pH = 11.

5. Procédé selon l'une ou plusieurs parmi les revendications 1 à 4, **caractérisé en ce que** l'étape b) est effectuée sans régulation de pH après ajustement préalable du pH de la suspension de l'oxyde de métal à une valeur de pH = 7 à pH = 11, et le pH est ensuite abaissé jusqu'à de pH = 5 à pH = 8.

6. Procédé selon l'une ou plusieurs parmi les revendications 1 à 5, **caractérisé en ce que** l'étape b) est effectuée à une température élevée, préférablement à une température dans la plage allant de 50°C à 100°C.

Fig. 1:

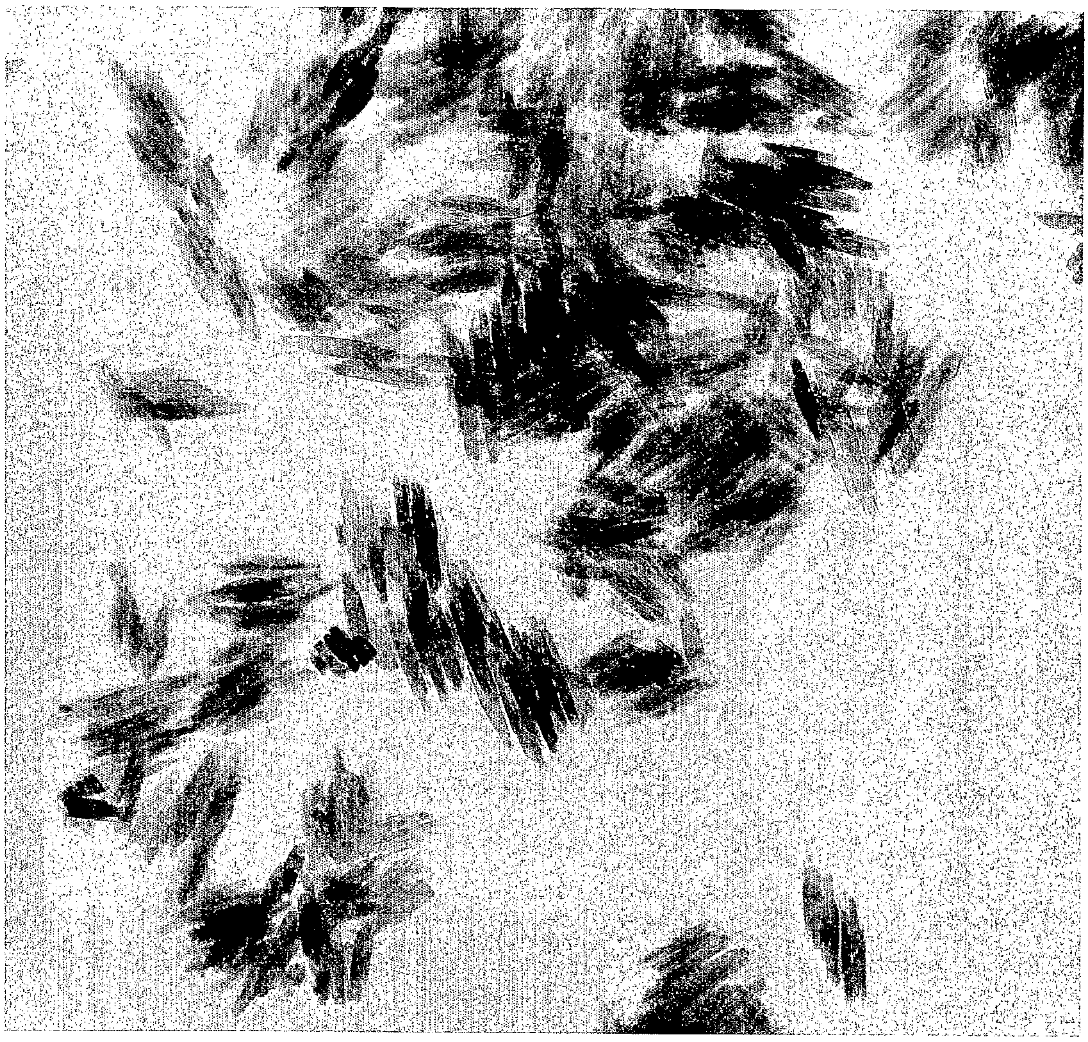

Fig. 2:

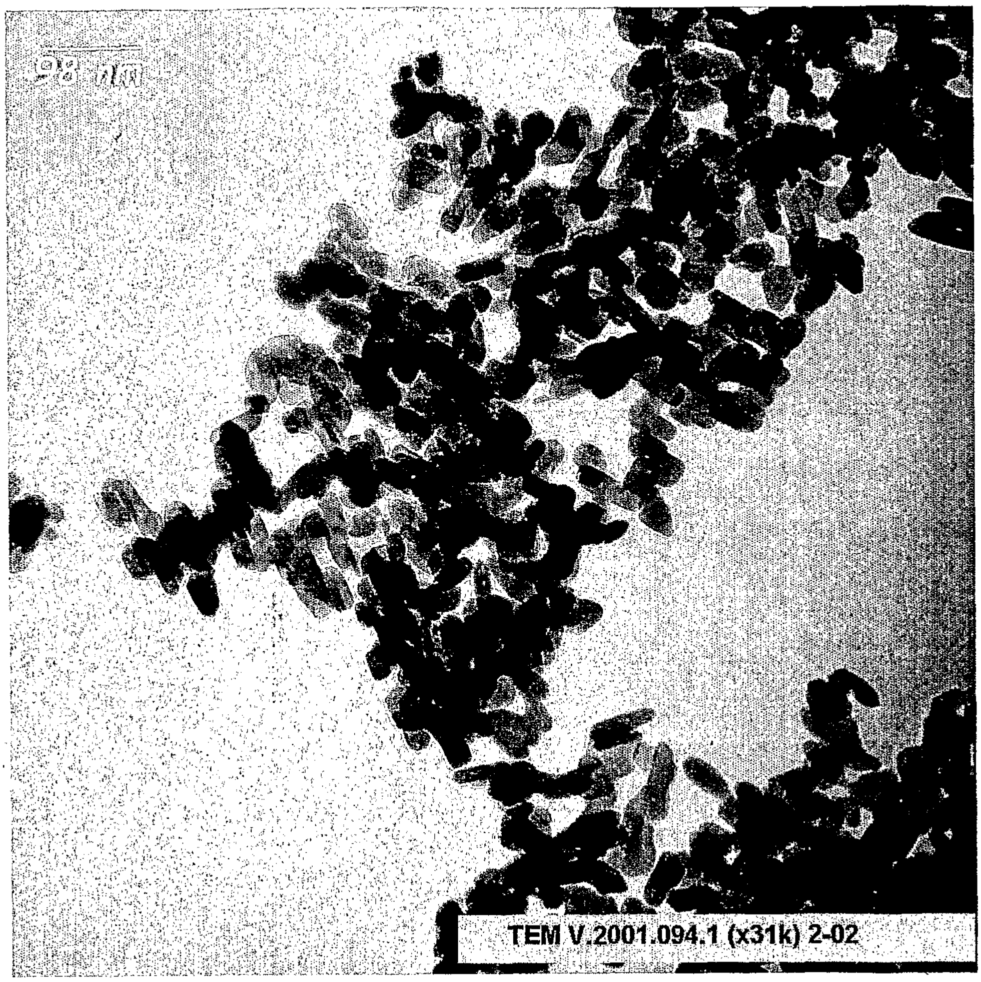

Fig. 3

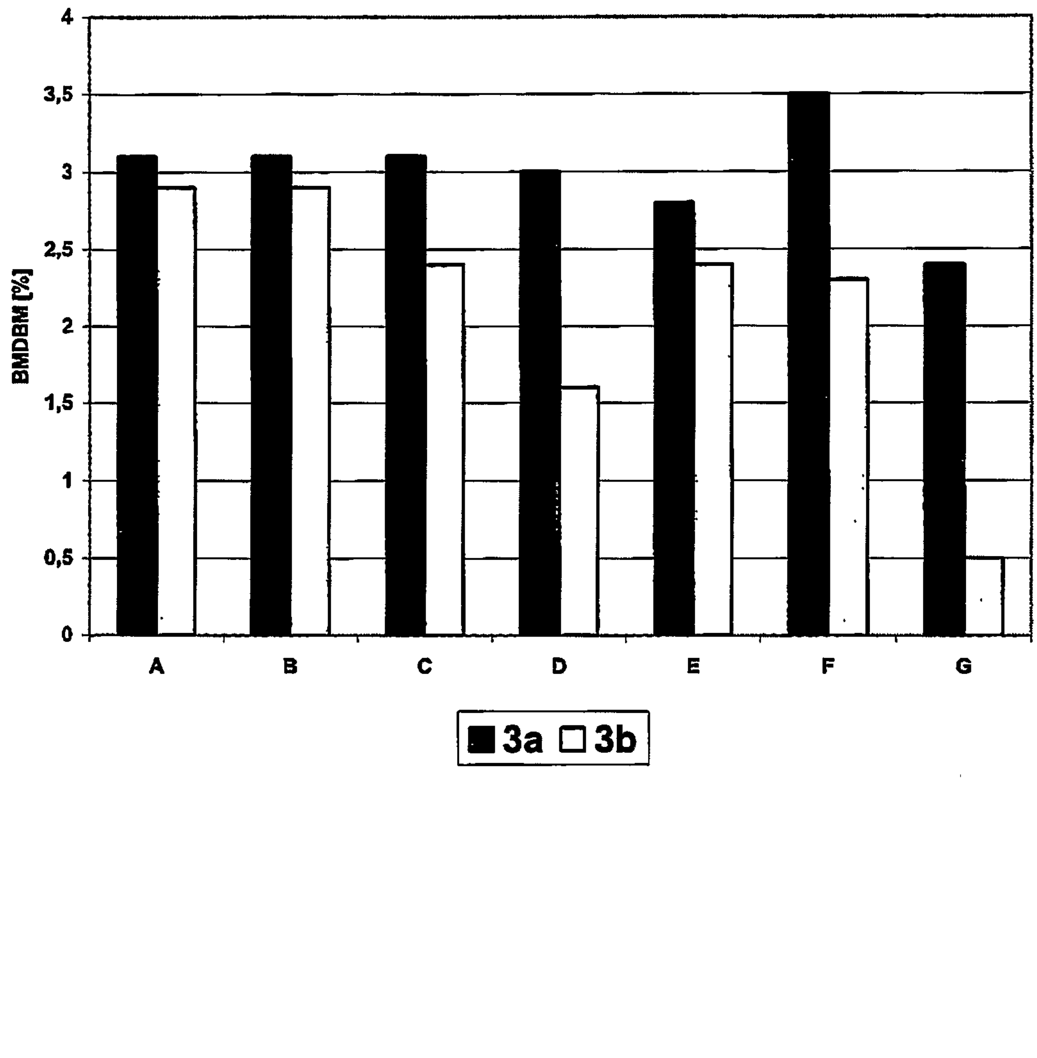

Fig. 4

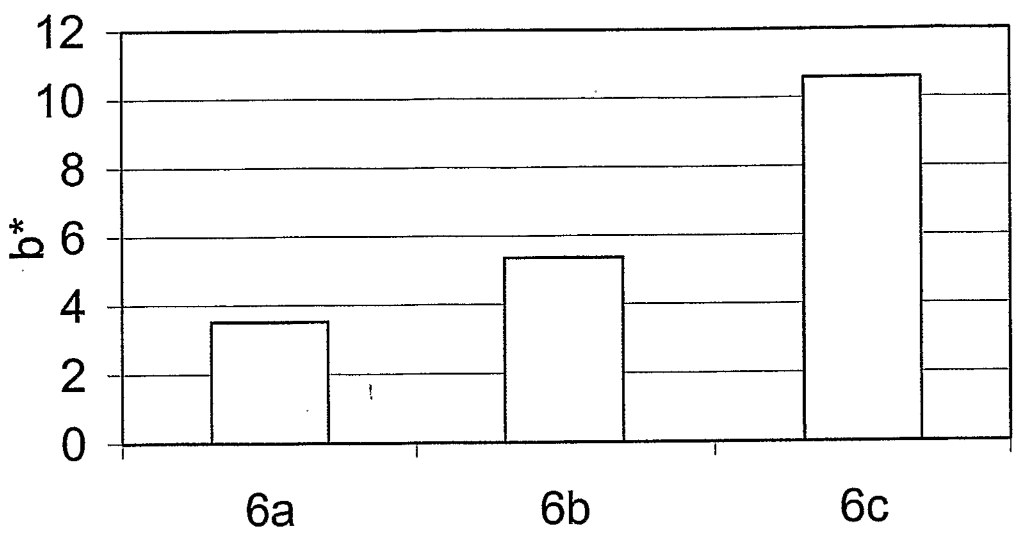

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- FR 2326405 A
- FR 2440933 A
- EP 0114607 A
- JP 61215314 A
- EP 0748624 A
- WO 9404131 A
- EP 1287807 A
- EP 1284277 A
- EP 0988853 A
- US 2002017221 A
- WO 02068544 A
- EP 0595471 A
- US 2885366 A
- WO 9304665 A
- EP 0487404 A
- WO 9966896 A
- US 6242099 B1
- WO 0009652 A
- WO 0072806 A
- WO 0071084 A
- EP 0671161 A
- DE 4116123 A
- WO 03007906 A
- DE OS4308282 A


### In der Beschreibung aufgeführte Nicht-Patentliteratur


- **K.RECKER.** Ullmanns Enzyklopädie der Technischen Chemie. 1978, vol. 15, 117 ff
- Rowe Colour Index. Society of Dyers and Colourists, 1971
- **E. A. GALINSKI et al.** *Eur. J. Biochem.,* 1985, vol. 149, 135-139